# EUROPEAN PATENT SPECIFICATION

(11) **EP 4 196 019 B1**
(45) Date of publication and mention of the grant of the patent: **22.04.2026**
(21) Application number: 21856863.2
(22) Date of filing: 16.08.2021
(51) Int. Cl.: A61K 49/04, A61L 24/00

(54) **METHODS AND MATERIALS FOR EMBOLIZATION**
VERFAHREN UND MATERIALIEN ZUR EMBOLISATION
PROCÉDÉS ET MATÉRIAUX D'EMBOLISATION

(30) Priority: 14.08.2020 US 202063065880 P
(43) Date of publication of application: 21.06.2023
(73) Proprietor: The Regents of University of California, Oakland, CA 94607-5200 (US); Mayo Foundation For Medical Education And Research, Rochester, MN 55904 (US); Boston Scientific Scimed Inc., Maple Grove, Minnesota 55311-1566 (US)
(72) Inventor: OKLU, Rahmi, Chandler, Arizona 85249 (US); KHADEMHOSSEINI, Alireza, Los Angeles, California 90049 (US); JABBARZADEH, Ehsan, Columbia, South Carolina 29201 (US)
(74) Representative: Vossius & Partner Patentanwälte Rechtsanwälte mbB
(86) International application number: PCT/US2021/046154
(87) International publication number: WO 2022/036322

(56) References cited:
- WO-A1-2020/047394
- WO-A1-2020/198504
- US-A1- 2005 025 707
- US-A1- 2016 144 068
- US-A1- 2016 331 380
- US-A1- 2018 360 920
- US-A1- 2020 246 502
- AVERY REGINALD K. ET AL: "An injectable shear-thinning biomaterial for endovascular embolization", SCIENCE TRANSLATIONAL MEDICINE, vol. 8, no. 365, 16 November 2016 (2016-11-16), pages 365ra15, XP093041993, ISSN: 1946-6234, DOI: 10.1126/scitranslmed.aah5533
- ALBADAWI HASSAN, IZZET ALTUN, JINGJIE HU, ZEFU ZHANG, ANSHUMAN PANDA, HAN-JUN KIM, ALI KHADEMHOSSEINI, RAHMI OKLU: "Nanocomposite Hydrogel with Tantalum Microparticles for Rapid Endovascular Hemostasis", ADVANCED NANOBIOMED RESEARCH, vol. 8, no. 1, 30 November 2020 (2020-11-30), XP055908686, DOI: 10.1002/advs.202003327
- RAJABI ET AL.: "An adhesive and injectable nanocomposite hydrogel of thiolated gelatin/gelatin methacrylate/Laponite as a potential surgical sealant", JOURNAL OF COLLOID AND INTERFACE SCIENCE, vol. 564, 22 March 2020 (2020-03-22), pages 155 - 169, XP085998231, DOI: 10.1016/j.jcis.2019.12.048
- NASROLLAHI FATEMEH, NAZIR FARZANA, TAVAFOGHI MARYAM, HOSSEINI VAHID, ALI DARABI MOHAMMAD, PARAMELLE DAVID, KHADEMHOSSEINI ALI, AHA: "Graphene Quantum Dots for Fluorescent Labeling of Gelatin‐Based Shear‐Thinning Hydrogels", ADVANCED NANOBIOMED RESEARCH, vol. 1, no. 7, 1 July 2021 (2021-07-01), pages 2000113, XP055908689, ISSN: 2699-9307, DOI: 10.1002/anbr.202000113

## Description

### Cross-Reference To Related Applications

This application claims the benefit under 35 U.S.C. Section 119(e) of co-pending and commonly-assigned U.S. Provisional Patent Application Serial No 63/065,880, filed on August 14, 2020, and entitled "METHODS AND MATERIALS FOR EMBOLIZATION".

### STATEMENT REGARDING FEDERAL FUNDING

This invention was made with government support under HL137193 and HL140951 awarded by the National Institutes of Health. The government has certain rights in the invention.

### TECHNICAL FIELD

This disclosure relates to hydrogel compositions for use in a method of embolization of one or more blood vessels (e.g., one or more arteries) within a mammal (e.g., a human), and said hydrogel composition comprising gelatin, a nanosilicate and a radiopaque contrast agent, wherein said nanosilicate is a silicate nanoplatelet, and wherein said radiopaque contrast agent comprises tantalum particles.

### BACKGROUND INFORMATION

Hemorrhage in a setting of anticoagulation (ACA) is a serious medical emergency associated with high morbidity and mortality. In the US alone, there are over 2.6 million people with atrial fibrillation on ACA (Lloyd-Jones et al., Circulation, 121:948 (2010)). More than 30 million prescriptions for coumadin are written annually, and prescriptions for newer oral anticoagulants are also increasing; the bleeding frequency for coumadin alone is estimated to be 15-20 percent per year (Wysowski et al., Arch. Intern. Med., 167:1414 (2007); and Zareh et al., West. J. Emerg. Med., 12:386 (2011)). Bleeding risk is even higher in patients with mechanical valves and cardiac assist devices (CAD) (Eckman et al., Circulation, 125:3038 (2012); Kirley et al., Circ. Cardiovasc. Qual. Outcomes, 5:615 (2012); and Smith et al., J. Thorac. Dis., 7:2112 (2015)).

Introduced 15 years ago, liquid embolics are specially formulated materials designed to self-solidify upon deployment *in situ.* Once injected, liquid embolics undergo a transition to form a solid based on physicochemical mechanisms, including polymerization, precipitation and cross-linking through ionic, covalent, or thermal processes. Howeer, liquid embolics are associated with risks such as catheter entrapment (Qureshi et al., J. Vasc. Interv. Neurol., 8:37 (2015)), recanalization rates of up to 36% (Cekirge et al., Neuroradiology, 48:113 (2006)), and leakage during injection that can cause non-target embolization, angiotoxicity, and/or necrosis.

Reginald K. Avery et al., "An injectable shear-thinning biomaterial for endovascular embolization", Sci. Transl. Med.8,365ra156-365ra156(2016) describes a shear-thinning biomaterial in the form of a nanocomposite hydrogel containing gelatin and silicate nanoplatelets (Laponite XLG). To visualize the biomaterial it was mixed with Visipaque. The biomaterial can function as an embolic agent for endovascular embolization procedures.

### SUMMARY

The scope of this invention is defined by the claims. Embodiments in the description relating to methods of treatment are not covered by the claims. Instead any references in the description to methods of treatment refer to the compositions of the present invention for use in a method for treatment of the human (or animal) body by therapy (or for diagnosis). Any "embodiment" or "example" which is disclosed in the description but is not covered by the claims should be considered as presented for illustrative purpose only.

This disclosure provides hydrogel compositions for use in embolization (e.g., reversible embolization) of one or more blood vessels (e.g., one or more arteries) within a mammal (e.g., a human).

As demonstrated herein, a radiopaque hydrogel composition (e.g., a gel embolic material (GEM)) including 18% (w/v) Type A gelatin, 9% (w/v) silicate nanoplatelets, and 20% (w/w) tantalum particles) can be rapidly delivered into diseased blood vessels (e.g., vein or arteries) using clinical catheters to achieve embolization of first-order arteries such as the renal artery and iliac artery. Also as demonstrated herein, embolization using a radiopaque GEM including 18% (w/v) Type A gelatin, 9% (w/v) silicate nanoplatelets, and 20% (w/w) tantalum particles can be visualized *in vivo* using multiple imaging platforms.

Having the ability to perform embolization within a blood vessel of a mammal (e.g., a human) by delivering a hydrogel composition including gelatin, nanosilicates, and one or more radiopaque contrast agents provides a unique and unrealized opportunity to safely and quickly achieve hemostasis of one or more blood vessels within a mammal that can be visualized and monitored (e.g., during and/or following delivery). For example, a hydrogel composition including gelatin, nanosilicates, and one or more radiopaque contrast agents can be used to treat bleeding such as hemorrhage (e.g., hemorrhage of an internal organ). For example, using clinical catheters to deliver a hydrogel composition provided herein for embolization that is efficient, effective, safe, and/or cost-effective.

In general, the present invention relates to a hydrogel composition comprising: (a) gelatin; (b) a nanosilicate; and (c) a radiopaque contrast agent, wherein said nanosilicate is a silicate nanoplatelet, and wherein said radiopaque contrast agent comprises tantalum particles. The hydrogel composition can include from about 0.1 % (w/v) to about 20 % (w/v) of the gelatin. The hydrogel composition can include 18% (w/v) of the gelatin. The gelatin can be a Type A gelatin. The hydrogel composition can include from about 3 % (w/v) to about 9 % (w/v) of the nanosilicate. The hydrogel composition can include about 9% (w/v) of the nanosilicate. The nanosilicate is a silicate nanoplatelet. The hydrogel composition can include from about 2 % (w/w) to about 30 % (w/w) of the radiopaque contrast agent. The hydrogel composition can include about 20% (w/w) of the radiopaque contrast agent, and the radiopaque contrast agent includes tantalum particles. The viscosity of the hydrogel composition can decrease under a shear rate of about 10⁻¹ 1/s. The hydrogel composition can have a displacement pressure of from about 15 kPa to about 45 kPa.

Also disclosed herein is this hydrogel composition for use in methods for embolization of a blood vessel within a mammal. The methods can include, or consist essentially of, delivering a hydrogel composition including gelatin, a nanosilicate, and a radiopaque contrast agent to a blood vessel within a mammal. The mammal can be a human. The delivery can include catheter-directed delivery. The delivery can include a delivery rate of from about 1 mL/minute to about 2 mL/minute. The delivery can include from about 2 mL to about 5 mL of said hydrogel composition.

Also disclosed herein is this hydrogel composition for use in methods for reducing blood flow in a blood vessel within a mammal. The methods can include, or consist essentially of, delivering a hydrogel composition including gelatin, a nanosilicate, and a radiopaque contrast agent to a blood vessel within a mammal. The mammal can be a human. The delivery can include catheter-directed delivery. The delivery can include a delivery rate of from about 1 mL/minute to about 2 mL/minute. The delivery can include from about 2 mL to about 5 mL of said hydrogel composition.

Also disclosed herein is this hydrogel composition for use in methods for inducing clotting in a blood vessel within a mammal. The methods can include, or consist essentially of, delivering a hydrogel composition including gelatin, a nanosilicate, and a radiopaque contrast agent to a blood vessel within a mammal. The clotting can be induced in less than about 20 minutes following the delivery. The clotting can include the formation of a blot clot having a volume of from about 0.5 cubic centimeters (cm³) to about 5 cm³. The mammal can be a human. The delivery can include catheter-directed delivery. The delivery can include a delivery rate of from about 1 mL/minute to about 2 mL/minute. The delivery can include from about 2 mL to about 5 mL of said hydrogel composition.

Also disclosed herein is this hydrogel composition for use in methods for treating a mammal having a bleeding disorder. The methods can include, or consist essentially of, delivering a hydrogel composition including gelatin, a nanosilicate, and a radiopaque contrast agent to a blood vessel within a mammal. The bleeding disorder can be non-traumatic hemorrhage, traumatic hemorrhage, a saccular aneurysm, a vascular malformation, an endoleak, a gastroesophageal varices, or an arteriovenous fistula. The mammal can be a human. The delivery can include catheter-directed delivery. The delivery can include a delivery rate of from about 1 mL/minute to about 2 mL/minute. The delivery can include from about 2 mL to about 5 mL of said hydrogel composition.

Also disclosed herein is this hydrogel composition for use in methods for treating a mammal having a tumor. The methods can include, or consist essentially of, delivering a hydrogel composition including gelatin, a nanosilicate, and a radiopaque contrast agent to a blood vessel within a mammal that is feeding a tumor within the mammal. The tumor can be a benign tumor. The tumor can be a malignant tumor. The tumor can be a hepatic tumor, a uterine fibroid, a prostate tumor, a renal tumor, or a cerebral tumor. The mammal can be a human. The delivery can include catheter-directed delivery. The delivery can include a delivery rate of from about 1 mL/minute to about 2 mL/minute. The delivery can include from about 2 mL to about 5 mL of said hydrogel composition.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF THE DRAWINGS

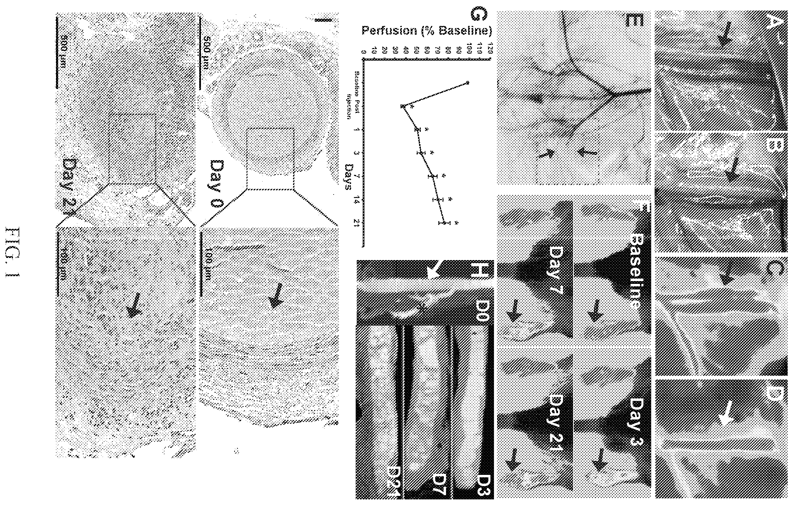

Figures 1A-1I. The rat model of femoral artery embolization using GEM. Images of the rat femoral artery (FA; arrow) before (Figure 1A) and after (Figure 1B, arrow) GEM injection. Laser speckle contrast microperfusion imaging (LSCI) of exposed FA before (Figure 1C, arrow) and after (Figure 1D, arrow) GEM injection showing the absence of blood flow. Figure 1E. Digital subtraction angiography from a catheter in the distal aorta shows GEM occlusion of the right femoral artery (arrows indicate collateral arteries that bypass the GEM occlusion). Figure 1F. A panel of representative hind limb microperfusion images using LSCI at baseline, day 3, day 7, and day 21 following GEM embolization of FA (arrow); contralateral limb is the untreated control. Figure 1G. Quantitative analysis of LSCI hind limb perfusion at baseline, post-GEM embolization at day 0, 1, 3, 7, 14, and 21 days (* p< 0.0001 compared to baseline; n=6). Figure 1H. Reconstructed maximum intensity projection (MIP) image of FA showing GEM with contrast agent within the lumen of the embolized artery (arrow) at day 0, 3, 7 and 21 days; these show time-dependent structural changes of GEM inside the artery suggesting progressive material remodeling (* at day 0 indicates GEM in the lateral circumflex branch artery). Figure 1I. H&E stained cross-sections of GEM embolized FA at day 0 and day 21; arrow at day 0 indicates complete occlusion of the arterial lumen with GEM. The arrow at day 21 indicates remodeling with near-complete replacement of GEM with granulation tissue inside the lumen of FA.

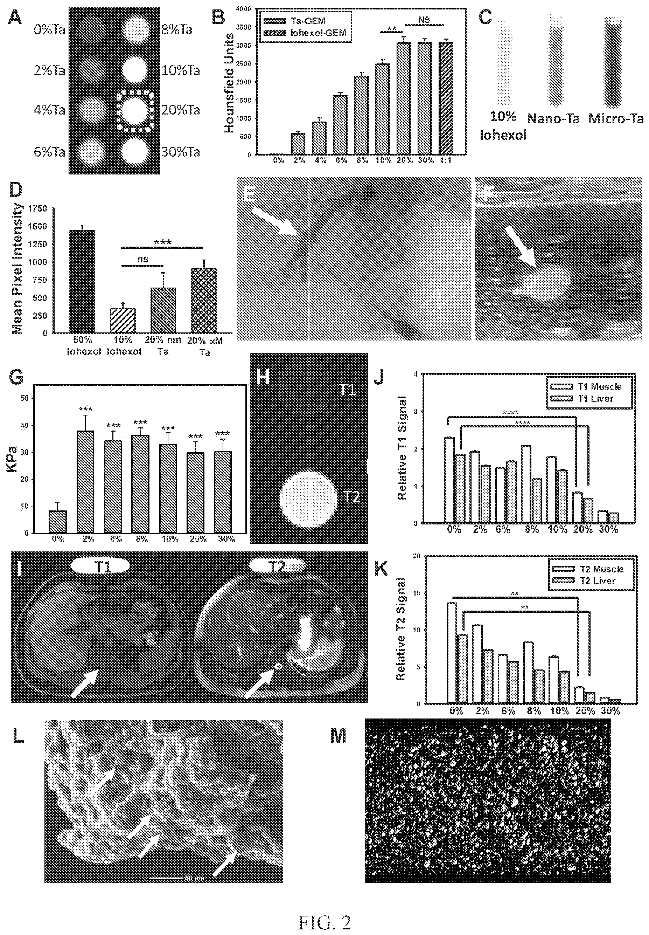

Figures 2A-2M. Assessing GEM visibility with multiple imaging modalities. Figure 2A. CT axial images of 8 syringes loaded with 0-30%Ta-GEM inside a phantom simulating the human chest are shown; 20%Ta GEM (dashed outline) demonstrates similar Hounsfield units (Figure 2B) when compared to 30%Ta GEM or 50% Iohexol GEM (n=4). Figure 2C. Fluoroscopic images of syringes loaded with GEM containing 10%-Iohexol, 20% Ta nanoparticles (Nano-Ta), or 20% Ta microparticles (Micro-Ta); quantitation of the mean pixel intensity on fluoroscopy images show 20% micro-Ta produce the highest intensity shown in (Figure 2D; n=4). Figure 2E - Figure 2F. Respective ultrasound and fluoroscopic images during percutaneous hepatic vein embolization in pig using 20%Ta-GEM (white arrow). Figure 2G. Graphic summary of shear wave elastography of GEM containing 0-30% Ta microparticles (n=3). Figure 2H. Transverse view of standard T1 and T2 MR imaging of syringe filled with 20%Ta-GEM showing attenuation for T1-sequence and bright signal for T2-sequence. Figure 2I. T1 and T2 signal of muscle and liver was calculated relative to an MRI phantom. Figure 2J and Figure 2K. The T1 and T2 signal of 20% Ta GEM relative to the signal calculated (Figure 2I) is depicted in the graphs; these indicate that the signal intensity is greater than muscle or liver suggesting that 20% Ta GEM will be visible by MRI. Figure 2L. Scanning electron microscopy of 20%Ta-GEM (white arrows, tantalum microparticles). Figure 2M. Micro-CT image of 20%Ta-GEM loaded syringe showing uniform distribution of Ta in GEM. Data are mean ± SD (***P* <0.01, ****P* <0.001, ****P <0.0001).

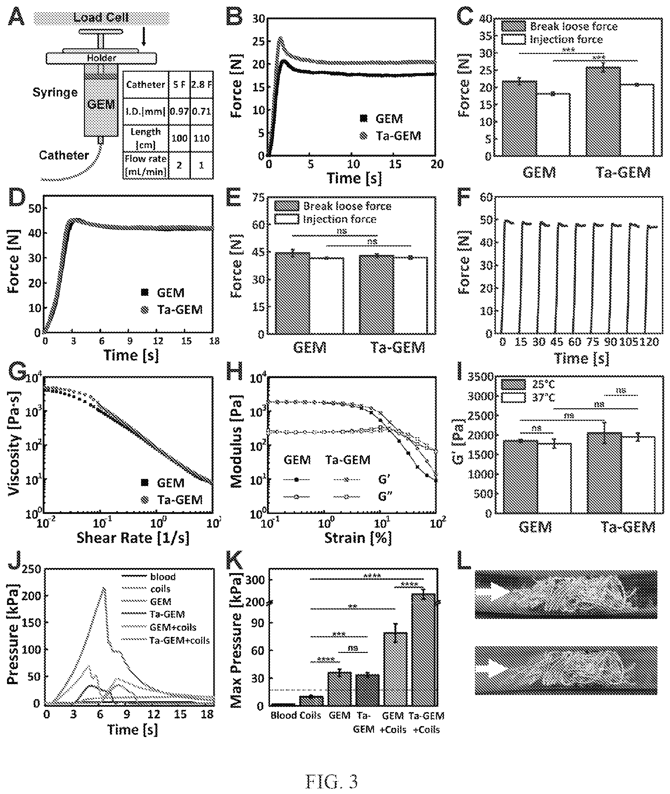

Figures 3A-3L. Physical and mechanical characterization of Ta-GEM. Figure 3A. Schematic of injection force testing. Table indicates the catheters and the parameters used during testing (I.D.: inner diameter of the catheter). Figure 3B. Representative injection force curves of GEM and Ta-GEM in a 1mL syringe injected through 110 cm, 2.8F microcatheter at 1 mL/minute. Figure 3C. Summary of break loose force and injection force of GEM and Ta-GEM in a 2.8F microcatheter system (n=5). Figure 3D. Representative injection force curves of GEM and Ta-GEM, respectively, loaded in a 3mL syringe and injected through 100 cm, 5F catheter at 2 mL/minute. Figure 3E. Summary of break loose force and injection force of GEM and Ta-GEM in a 5F catheter system (n=5). Figure 3F. Injection force profile of an interrupted delivery (10 second delivery, followed by 5 second dwell time) of Ta-GEM through a 5F catheter at 2 mL/minute. Figure 3G. Flow curves comparing GEM and Ta-GEM at 37 °C showing shear thinning properties. Figure 3H. Amplitude sweeps of GEM and Ta-GEM at 37 °C. Figure 3I. Summary of storage modulus kinetics of GEM and Ta-GEM at 25 °C and 37 °C (n=3). Data are mean ± SD for Figure 3C, Figure 3E, and Figure 3I. Figure 3J. Resistance pressure curves in *in vitro* embolization tests. Figure 3K. Graphic summary showing peak pressure is maximally generated by Ta-GEM + Coils compared to coil alone (** p< 0.01, ***p< = 0.001, ****p<0.0001, data represents the mean ± SD, with n=3). Figure 3L. Photograph of coil fibers inside the vascular model following catheter delivery of GEM (shown in green) before and after injection.

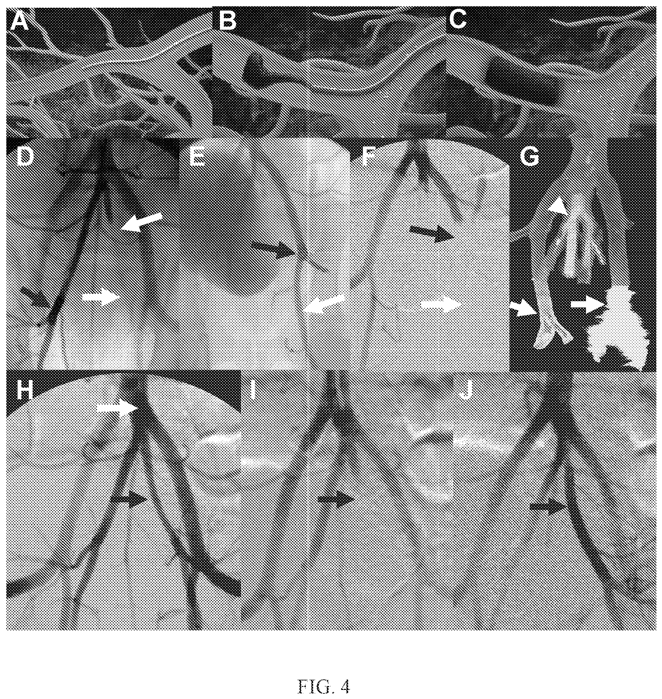

Figures 4A-4J. Assessing Ta-GEM embolization in the anticoagulated swine iliac artery. Figure 4A - Figure 4C. Schematic of transcatheter arterial embolization with Ta-GEM. Figure 4D. Digital subtraction angiography (DSA) showing the failure of coils to achieve occlusion (black arrow; contrast flows through the coil mass inside the artery) and white arrows in the contralateral iliac artery showing complete occlusion of the artery with Ta-GEM. Figure 4E. Contrast injection in the iliac artery shows contrast flowing through the coil mass (black arrow) and beyond (white arrow). Following the delivery of GEM to the coil mass in Figure 4E, a DSA image in Figure 4F shows complete occlusion with no break-through blood flow (arrows). Figure 4G. Coronal micro-CT image of Figure 4F shows GEM (arrowhead) uniformly casting the internal iliac arteries without artifact; in contrast, the coil mass (arrow) produced significant streak artifact limiting assessment of the artery and adjacent branch. Figure 4H - Figure 4J. Serial DSA images of the normal swine pelvic arteries in Figure 4H, Ta-GEM-embolized left internal iliac artery (arrow) in Figure 4I and DSA following complete retrieval of Ta-GEM in Figure 4J showing the restoration of blood flow in the artery. Retrieval of Ta-GEM was performed 3 hours after embolization of the artery.

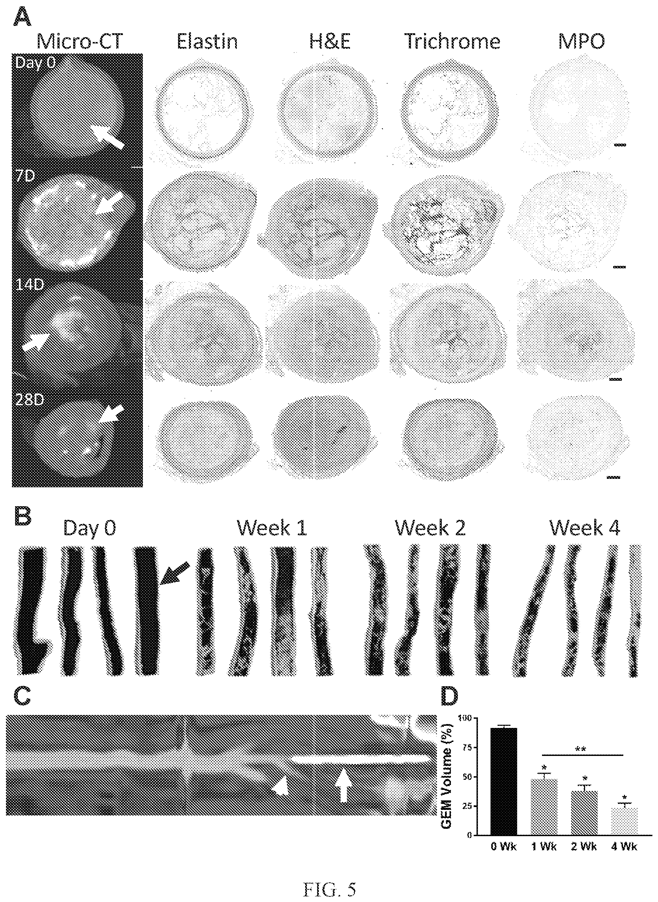

Figures 5A-5D. Assessing swine internal iliac artery occlusion computed tomography and histology. Figure 5A. A panel of micro-CT images and the corresponding cross-sections of the internal iliac artery on histology at 0, 1, 2, or 4 weeks following embolization stained with H&E, VG Elastin, Mason's trichrome and myeloperoxidase (MPO), respectively are shown. At Day 0, there is uniform occlusion of the artery with Ta-GEM (arrow). Subsequently, a concentric fibroinflammatory reaction ensues that leads to biodegradation of GEM. Figure 5B. 3D coronal rendering of reconstructed high-resolution micro-CT scans of the explanted pig internal iliac arteries following segmentation of Ta-GEM inside each vessel at 0, 1, 2, or 4 weeks after embolization (arrow points to GEM, black color inside artery; connective tissue is depicted as gray). Over time, there is progressive biodegradation of Ta-GEM and increase in fibrosis (gray). Figure 5C. Reconstructed linear depiction of the aorta to the iliac artery showing Ta-GEM inside the left internal iliac artery (arrow) and patent right internal iliac artery (arrowhead). Figure 5D. Quantitation of Ta-GEM over time from the microCT segmentation dataset in Figure 5B is shown as a graph revealing progressive decrease of GEM inside the artery. Data are the mean ± SEM with n=4, * p<0.0001, ** p=0.007 using ANOVA and Tukey's post hoc analysis.

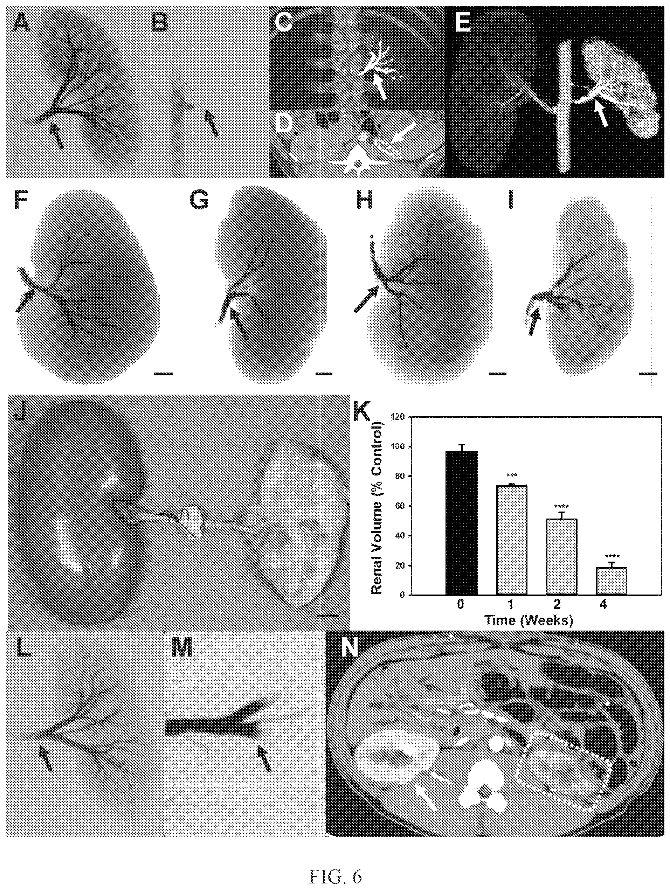

Figures 6A-6N. Swine renal artery embolization using Ta-GEM and comparison to gelfoam. Fluoroscopic images before (Figure 6A) and after (Figure 6B) renal artery embolization with Ta-GEM; following injection of 2-3 mL of Ta-GEM, there is complete absence of renal arterial flow to the kidney (arrow). Figure 6C. The maximum intensity projection image shows the left main renal artery and segmental branches occluded with Ta-GEM (arrow). Axial CT (Figure 6D) and 3D reconstructed image (Figure 6E) shows the markedly atrophic kidney with Ta-GEM in renal artery. Figure 6F - Figure 6I. Representative coronal CT images of embolized kidneys with Ta-GEM at 0, 1, 2, or 4 weeks, respectively. The cortex of the kidney is devoid of Ta-GEM. Figure 6J. Photograph showing the gross appearance of the embolized kidney at 4 weeks with compensatory hypertrophy of the contralateral kidney. Figure 6K. Summary of total kidney volume measurements following renal artery embolization (*** p<0.001, **** p<0.0001 using one-way analysis of variance, n=4 in each group). Over-time there is progressive atrophy of the kidney. DSA image before (Figure 6L) and after (Figure 6M) embolization of renal artery using gelfoam (black arrow). Figure 6N. At two weeks post-embolization, axial CT image shows enhancement of the embolized kidney and blood flow in the arteries despite successful renal artery embolization (dashed outline). Data reported as the mean ± SEM.

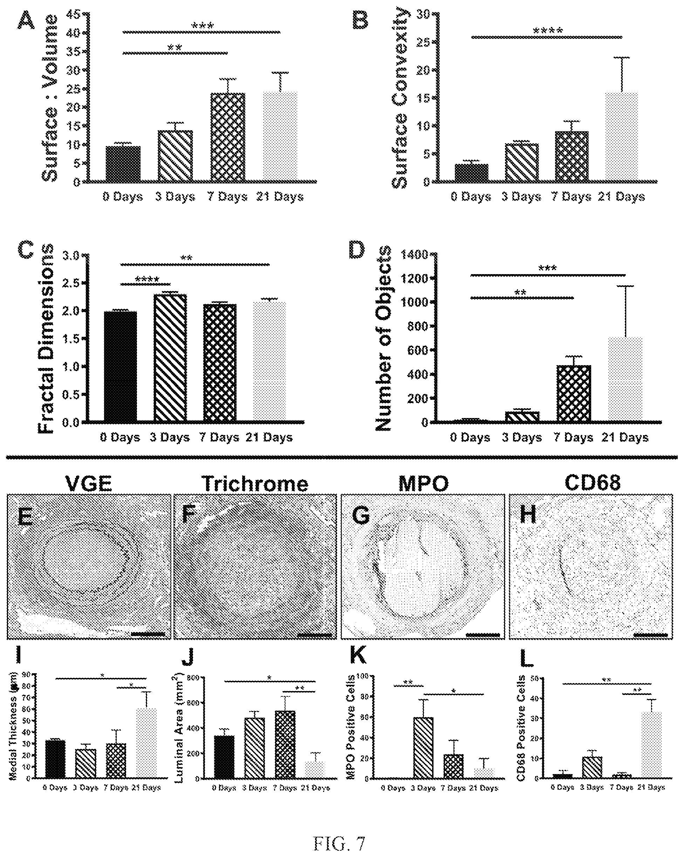

Figures 7A-7L. Time-dependent characterization of morphologic changes of GEM in the rat femoral artery using micro-CT and histology. A-D. Micro-CT analysis of surface to volume ratio, surface Convexity index, fractal dimensions, and structure model index, respectively, indicating structural changes of GEM inside the rat FA. Representative FA tissue sections stained with Verhoeff-Van Gieson (E), Mason's trichrome stain (F), myeloperoxidase (MPO) and CD68 (H). I-J. Graph of time-dependent change in medial thickness and luminal area at different time points following embolization. K. Graphic summary of the average number of MPO-expressing cells per high power field for each time point showing significant granulocytes infiltration at 3 days and decrease by 7 and 21 days. L. Graph summarizing the average number of CD68 expressing cells per high power demonstrating significant recruitment of macrophages at 21 days after GEM embolization. Data are mean ± SEM (*P <0.05, ***P* <0.01, ****P* <0.001, *****P* <0.0001; n=6). Scale, 200 µm.

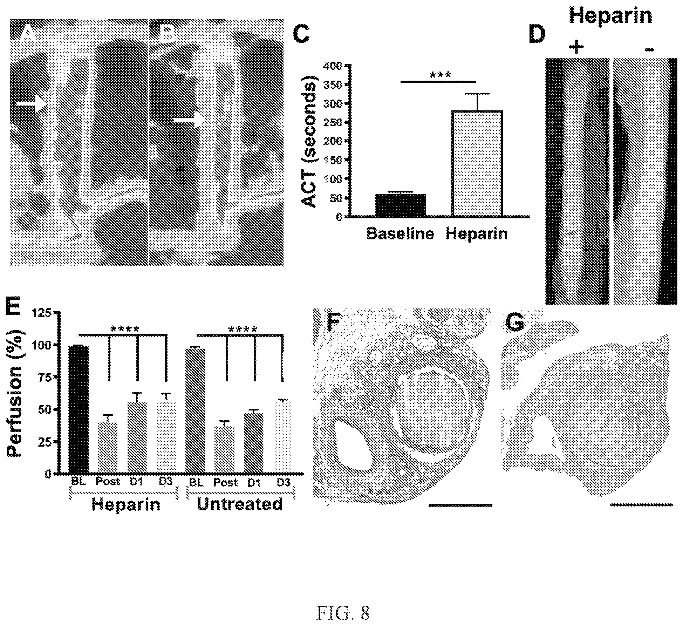

Figures 8A-8G. Rat femoral artery embolization in a state of anticoagulation. Laser speckle contrast imaging (LSCI) of femoral artery and vein in a heparinized rat showing patent femoral artery at baseline (A; arrow) and complete occlusion following embolization with GEM (B; white arrow). C. Graph showing significant increase in activated clotting time (ACT) following heparin injection compared to baseline confirming anticoagulation. D. Coronal micro-CT images of the explanted femoral artery with Ta-GEM from anticoagulated and control rats at 3 days after embolization. E. Quantitative analysis of hind limb perfusion using LSCI show no difference between anticoagulated and normal rats at 1 and 3 days after embolization suggesting no evidence for fragmentation or migration (n=6). F-G. Respective histologic micrographs of rat femoral artery cross sections obtained from anticoagulated or untreated rats at 3 days after embolization showing GEM persistently casting both vessel lumen. Data are mean ± SEM (****P* <0.001, *****P* <0.0001; n=6). Scale, 650 µm.

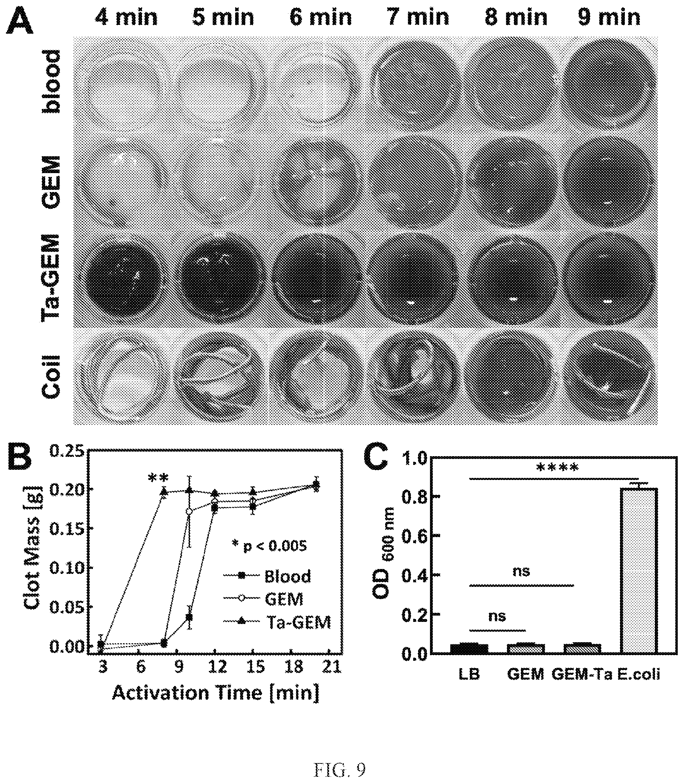

Figures 9A-9C. Evaluating the effect of tantalum microparticles on blood thrombogenicity and GEM sterility. A. Time to thrombosis of blood alone, GEM, Ta-GEM and a clinically used embolization coil mixed with blood is shown. GEM, Ta-GEM and coil samples demonstrated evidence of thrombosis as early as 4 min. B. Average mass of formed clot over time show enhanced thrombogenicity with Ta-GEM compared to blood alone or GEM alone. C. Graphic summary of sterility test of GEM and GEM-Ta following inoculation of LB broth and incubation for 24 hours at 37°C; these were compared to LB broth alone and LB broth inoculated with E Coli bacteria. Optical density was measured in each aliquot at 600 nm. Results are mean ± SEM (***P* <0.01, *****P* <0.0001; n=4).

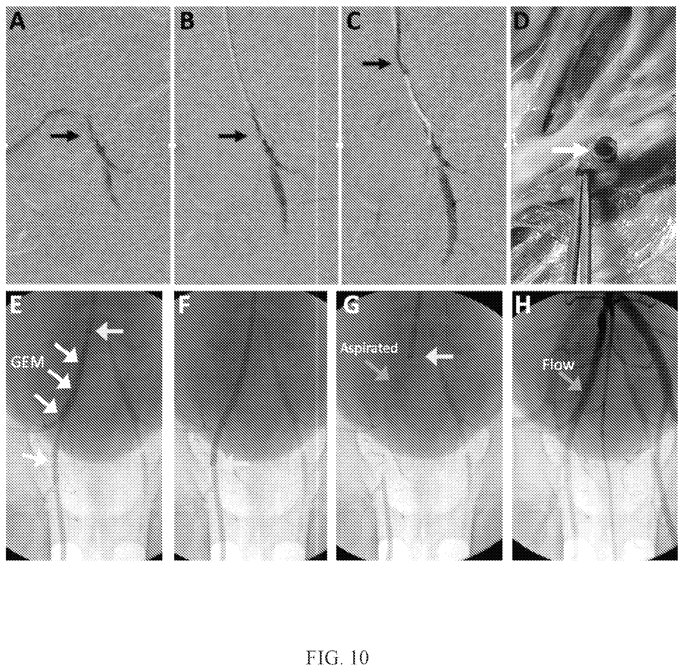

Figures 10A-10H. Catheter-directed delivery and retrieval of Ta-GEM from pig arteries. A-C. Serial digital subtraction angiography (DSA) images during Ta-GEM injection into iliac artery is shown (for real-time video, see Movie S1). These images demonstrate instant occlusion upon exit from the catheter without distal fragmentation. D. On necropsy, the iliac artery containing Ta-GEM is shown (arrow). E-H. Representative images of an internal iliac artery initially embolized with Ta-GEM (E; white arrow) and later retrieved using the Penumbra aspiration catheter ((F, G) yellow arrow). H. Shows restoration of flow following retrieval of GEM.

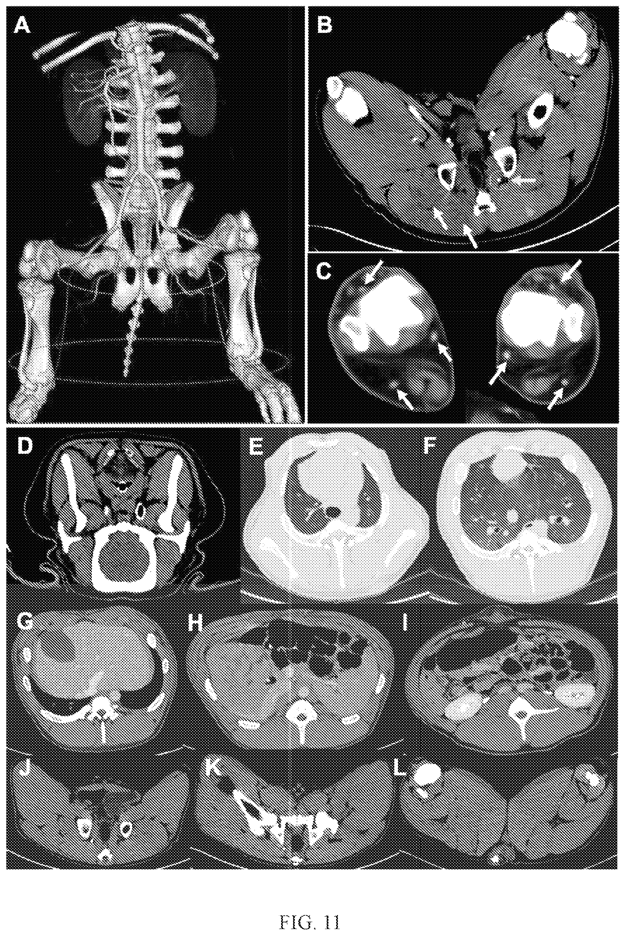

Figures 11A-11L. Assessing arterial occlusion in the pig and organ integrity with computed tomography angiography at 4 weeks post-embolization. A. Reconstructed 3D images from a contrast enhanced CT angiography shows absence of the right internal iliac artery (red arrow); this artery does not opacify because it is occluded with Ta-GEM. Axial image of the dotted yellow circle in (A) is displayed in (B). B. Red circle shows absence of flow in the embolized artery with geographic hypodensity in the gluteal muscles indicating evidence of ischemia (white arrow). Contralateral artery (small yellow circle) is widely patent with no evidence of hypodensity or ischemia in the respective gluteal muscles (blue arrowhead). Axial image of the dotted blue circle in (A) is displayed in (C). C. Axial image of the distal hindlimbs demonstrates normal run-off vessels (arrows) to the feet suggesting that there is no evidence for non-target embolization. Panel of axial CT images of the brain (D), heart (E), lungs (F), liver (G), spleen (H), kidneys (I), pelvis (J, K), hind limbs (L), reveal normal radiographic appearance.

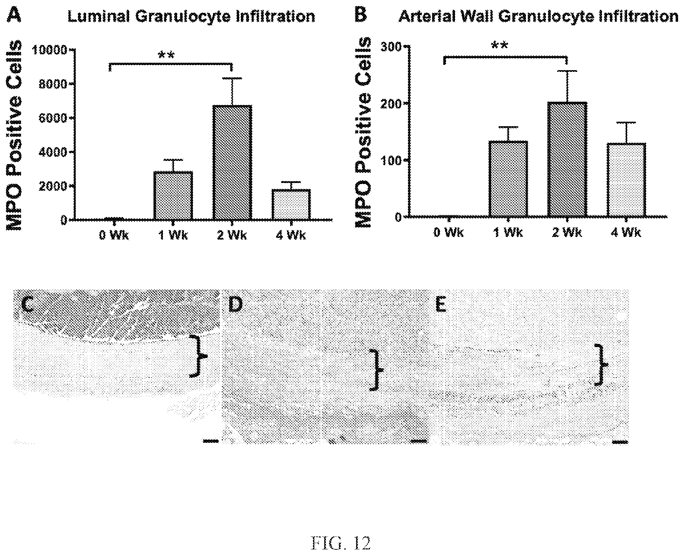

Figures 12A-12E. Quantitative analysis of inflammatory cell infiltration following internal iliac artery embolization and histology. A. Summary graph of the average number of MPO expressing cells inside the lumen of the pig's internal iliac artery embolized with Ta-GEM; there is significant infiltration of MPO expressing cells at 2 weeks following embolization (** p=0.004 using ANOVA with Kruskal-Wallis post hoc test. B. Graph of MPO expressing cells infiltrating the arterial wall compartment show a marked increase at 2 weeks following embolization. ** p=0.007 using ANOVA with Tukey's post hoc tests. Data are the mean ± SEM, n=4 for each data point. C. On histology, at day 0 following embolization, Trichrome stain demonstrates an intact media layer. D. At day 14, however, there is disruption and fibrosis of the media layer; E. Elastin stain of the media layer shows disruption of the intima and media layers. Scale bar 50 microns.

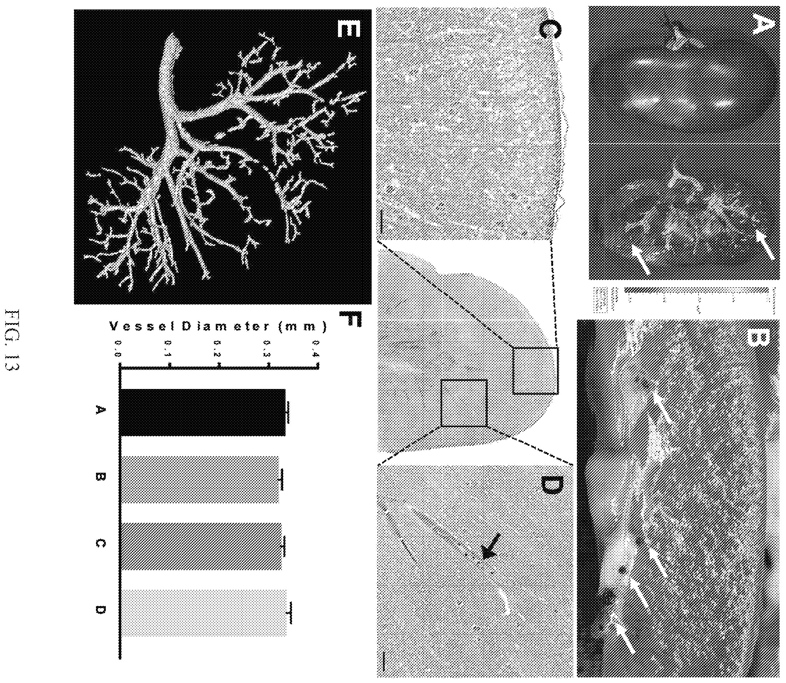

Figures 13A-13F. Assessing intravascular distribution of Ta-GEM following renal artery embolization in pigs using a Fogarty catheter. A. A 4 French Fogarty catheter in the mid-main renal artery was inflated and Ta-GEM was injected continuously until the balloon began to displace proximally. Following coronal transection of the kidney at necropsy, near-infrared imaging of this kidney embolized with Ta-GEM containing indocyanine green showed distribution of GEM in the main renal artery and in segmental arteries sparing the cortex. B. Transverse section across the inferior pole shows arterioles embolized with Ta-GEM (arrows). C. H&E of a slide showing the cortex demonstrates normal glomeruli with absence of Ta-GEM in the cortex. D. H&E image showing interlobar arterioles embolized with Ta-GEM. (scale; 500 µm). E. The whole kidney in (A) was imaged ex vivo in a microCT scanner; these images show embolic material in blood vessels reaching proximal arcuate arterioles. F. Graphic summary of arterial diameters measured in four kidneys reveal a mean vessel diameter of the smallest arteries embolized to be in the range of 320 µm.

### DETAILED DESCRIPTION

In the description of embodiments, reference may be made to the accompanying figures which form a part hereof, and in which is shown by way of illustration a specific embodiment in which the invention may be practiced. It is to be understood that other embodiments may be utilized, and structural changes may be made without departing from the scope of the present invention. Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. Many of the aspects of the techniques and procedures described or referenced herein are well understood and commonly employed by those skilled in the art. The following text discusses various embodiments of the invention.

This disclosure provides materials for use in embolization of one or more blood vessels (e.g., one or more arteries) within a mammal (e.g., a human). For example, this disclosure provides hydrogel compositions that can be delivered to one or more blood vessels (e.g., one or more arteries) within a mammal (e.g., a human) for embolization of the blood vessel(s). In some cases, one or more hydrogel compositions provided herein can be delivered to one or more blood vessels within a mammal (e.g., a human) to induce formation of a thrombus (e.g., an artificial embolus) within the blood vessel(s). In some cases, one or more hydrogel compositions provided herein can be delivered to one or more blood vessels within a mammal (e.g., a human) to form an embolus (e.g., an artificial embolus) within the blood vessel(s).

A hydrogel composition provided herein includes gelatin, one or more nanosilicates, and one or more radiopaque contrast agents, wherein said nanosilicate is a silicate nanoplatelet, and wherein said radiopaque contrast agent comprises tantalum particles. In some cases, a hydrogel composition provided herein can be sterile. In some cases, a hydrogel composition provided herein can be anti-bacterial. In some cases, a hydrogel composition provided herein can be bioactive. For example, a hydrogel composition provided herein can include one or more therapeutic agents.

A hydrogel composition provided herein can include any type of gelatin. In some cases, a hydrogel composition can include a single type of gelatin. In some cases, a hydrogel composition can include two or more (e.g., two, three, four, or more) types of gelatin. In some cases, a gelatin can be a synthetic gelatin. In some cases, a gelatin can be extracted from a tissue (e.g., skin, bone, and connective tissues) of an animal. Examples of types of gelatin that can be included in a hydrogel composition provided herein include, without limitation, Type A gelatin, Type B gelatin, gelatin extracted from cattle, gelatin extracted from pigs, and gelatin extracted from fish.

A hydrogel composition provided herein can include any amount of gelatin. For example, a hydrogel composition provided herein can include from about 0.1 % (w/v) to about 20 % (w/v) gelatin (e.g., from about 0.5 % (w/v) to about 20 % (w/v) gelatin, from about 1 % (w/v) to about 20 % (w/v) gelatin, from about 5 % (w/v) to about 20 % (w/v) gelatin, from about 10 % (w/v) to about 20 % (w/v) gelatin, from about 12 % (w/v) to about 20 % (w/v) gelatin, from about 15 % (w/v) to about 20 % (w/v) gelatin, from about 17 % (w/v) to about 20 % (w/v) gelatin, from about 0.1 % (w/v) to about 18 % (w/v) gelatin, from about 0.1 % (w/v) to about 15 % (w/v) gelatin, from about 0.1 % (w/v) to about 12 % (w/v) gelatin, from about 0.1 % (w/v) to about 10 % (w/v) gelatin, from about 0.1 % (w/v) to about 8 % (w/v) gelatin, from about 0.1 % (w/v) to about 5 % (w/v) gelatin, from about 0.5 % (w/v) to about 18 % (w/v) gelatin, from about 1 % (w/v) to about 15 % (w/v) gelatin, from about 5 % (w/v) to about 12 % (w/v) gelatin, from about 8 % (w/v) to about 10 % (w/v) gelatin, from about 1 % (w/v) to about 5 % (w/v) gelatin, from about 5 % (w/v) to about 10 % (w/v) gelatin, or from about 10 % (w/v) to about 15 % (w/v) gelatin). In some cases, a hydrogel composition provided herein can include about 18% (w/v) gelatin (e.g., Type A gelatin). In some embodiments, the hydrogels of the present disclosure comprise from about 0.2 % to about 1.2 % of gelatin (w/w), including about 0.2 %, about 0.3 %, about 0.4 %, about 0.5 %, about 0.6 %, about 0.7 %, about 0.8 %, about 0.9 %, about 1.0 %, about 1.1 %, and about 1.2 %, including all values and ranges therebetween. In some embodiments, the hydrogels of the present disclosure comprise from about 0.6 % to about 1.0 % of gelatin (w/w), In some embodiments, the hydrogels of the present disclosure comprise from about 0.4 % to about 0.8 % of gelatin (w/w), In some embodiments, the hydrogels of the present disclosure comprise about 0.2 %, about 0.3 %, about 0.4 %, about 0.5 %, about 0.6 %, about 0.7 %, about 0.8 %, about 0.9 %, about 1.0 %, about 1.1 %, or about 1.2 % of gelatin (w/w). In some embodiments, the hydrogels of the present disclosure comprise about 0.6 % of gelatin (w/w). In some embodiments, the hydrogels of the present disclosure comprise about 0.8 % of gelatin (w/w).

A hydrogel composition provided herein can include a single type of nanosilicate in the form of silicate nanoplatelets. In some cases, a hydrogel composition can include two or more (e.g., two, three, four, or more) types of nanosilicates. Examples of nanosilicates that can be included in a hydrogel composition provided herein include, without limitation, lithium magnesium sodium silicates such as Laponite^{®}-based silicate nanoplatelets (e.g., Laponite^{®} XLG-based silicate nanoplatelets, Laponite^{®} XLS-based silicate nanoplatelets, Laponite^{®} XL21-based silicate nanoplatelets, and Laponite^{®} D-based silicate nanoplatelets).

A hydrogel composition provided herein can include any amount of nanosilicates. For example, a hydrogel composition provided herein can include from about 3 % (w/v) to about 9 % (w/v) nanosilicates (e.g., from about 4 % (w/v) to about 9 % (w/v) nanosilicates, from about 5 % (w/v) to about 9 % (w/v) nanosilicates, from about 6 % (w/v) to about 9 % (w/v) nanosilicates, from about 7 % (w/v) to about 9 % (w/v) nanosilicates, from about 8 % (w/v) to about 9 % (w/v) nanosilicates, from about 3 % (w/v) to about 8 % (w/v) nanosilicates, from about 3 % (w/v) to about 7 % (w/v) nanosilicates, from about 3 % (w/v) to about 6 % (w/v) nanosilicates, from about 3 % (w/v) to about 5 % (w/v) nanosilicates, from about 3 % (w/v) to about 4 % (w/v) nanosilicates, from about 4 % (w/v) to about 8 % (w/v) nanosilicates, from about 5 % (w/v) to about 7 % (w/v) nanosilicates, from about 3 % (w/v) to about 5 % (w/v) nanosilicates, from about 4 % (w/v) to about 6 % (w/v) nanosilicates, from about 5 % (w/v) to about 7 % (w/v) nanosilicates, or from about 6 % (w/v) to about 8 % (w/v) nanosilicates). In some cases, a hydrogel composition provided herein can include about 9% (w/v) nanosilicates (e.g., silicate nanoplatelets). In some embodiments, the hydrogels of the present disclosure comprise from about 2.5 % to about 6 % of nanosilicates (w/w), including about 2.5%, about 3.0 %, about 3.5%, about 4.0 %, about 4.5%, about 5.0 %, about 5.5%, and about 6.0 %, including all values and ranges therebetween. In some embodiments, the hydrogels comprise from about 3.0 % to about 4.0 % of nanosilicates (w/w). In some embodiments, the hydrogels comprise from about 3.25 % to about 3.75 % of nanosilicates (w/w). In some embodiments, the hydrogels comprise from about 4.0 % to about 5.5 % of nanosilicates (w/w). In some embodiments, the hydrogels comprise from about 4.25 % to about 5.25 % of nanosilicates (w/w). In some embodiments, the hydrogels comprise from about 4.5 % to about 5.0 % of nanosilicates (w/w). In some embodiments, the hydrogels comprise about 2.75 %, about 3.0 %, about 3.25 %, about 3.5 %, about 3.75 %, 4.0 %, about 4.25 %, about 4.5 %, about 4.75 %, about 5.0 %, about 5.25 %, or about 5.5 % of nanosilicates (w/w). In some embodiments, the hydrogels comprise about 4.75 % of nanosilicates (w/w). In some embodiments, the hydrogels comprise about 4 % of nanosilicates (w/w). In some embodiments, the hydrogels comprise about 3.5 % of nanosilicates (w/w).A hydrogel composition provided herein can have any ratio of gelatin to nanosilicates. For example, a hydrogel composition provided herein can have a ratio of gelatin to nanosilicates of from about 1:2 to about 1:9 (e.g., from about 1:2 to about 1:8, from about 1:2 to about 1:7, from about 1:2 to about 1:6, from about 1:2 to about 1:5, from about 1:2 to about 1:4, from about 1:3 to about 1:9, from about 1:4 to about 1:9, from about 1:5 to about 1:9, from about 1:6 to about 1:9, from about 1:7 to about 1:9, from about 1:3 to about 1:8, from about 1:4 to about 1:7, from about 1:5 to about 1:6, from about 1:2 to about 1:4, from about 1:3 to about 1:6, from about 1:4 to about 1:7, or from about 1:5 to about 1:8). In some cases, a hydrogel composition provided herein can have a ratio of gelatin to nanosilicates of about 1:6.

In some cases, a hydrogel composition provided herein can include water (e.g., ultrapure water). In some cases, a hydrogel composition provided herein can have a ratio of gelatin to nanosilicates to water of about 1:5:6. In some cases, a hydrogel composition provided herein can have a ratio of gelatin to nanosilicates to water of about 1:6:5.

A hydrogel composition provided herein can include a single type of radiopaque contrast agent in the form of tantalum particles. In some cases, a hydrogel composition can include two or more (e.g., two, three, four, or more) types of radiopaque contrast agents. Examples of further radiopaque contrast agents that can be included in a hydrogel composition provided herein include, without limitation, iodinated molecules (e.g., iohexol, iopromide, iodixanol, ioaglate, iothalamate, and iopamidol), gold nanoparticle (AuNPs), ethiodized oil (e.g., Lipiodol^{®}), lanthanide-based contrast agents, and bismuth. According to the claimed invention, the hydrogel composition includes tantalum particles. These tantalum particles can be any appropriate size of tantalum particles. In some cases, tantalum particles included in a hydrogel composition provided herein can be essentially the same size. In some cases, tantalum particles included in a hydrogel composition provided herein can have different sizes. Examples of tantalum particles that can be included in a hydrogel composition provided herein include, without limitation, tantalum microparticles (e.g., tantalum particles having an average size of about 2 µm), and tantalum nanoparticles (e.g., tantalum particles having an average size of about <25 nm). In some cases, a hydrogel composition provided herein can include tantalum particles having an average size of about 2 µm. In some cases, a hydrogel composition provided herein can include tantalum particles having an average size of about 30 µm. In some cases, a hydrogel composition provided herein can include tantalum particles having an average size of about 2 nm. In some embodiments, the hydrogels of the present disclosure comprise tantalum particles having a median particle size of about 1 µm to about 15 µm, including about 2 µm, 3 µm, 4 µm, 5 µm, 6 µm, 7 µm, 8 µm, 9 µm, 10 µm, 11 µm, 12 µm, 13 µm, and 14 µm and all values and ranges therebetween. In some embodiments, the hydrogels of the present disclosure comprise tantalum particles having a median particle size of about 2 µm to about 5 µm.

A hydrogel composition provided herein can include any amount of radiopaque contrast agent(s). For example, a hydrogel composition provided herein can include from about 2 % (w/w) to about 30 % (w/w) radiopaque contrast agent(s) (e.g., from about 2 % (w/w) to about 25 % (w/w), from about 2 % (w/w) to about 20 % (w/w), from about 2 % (w/w) to about 15 % (w/w), from about 2 % (w/w) to about 10 % (w/w), from about 2 % (w/w) to about 5 % (w/w), from about 5 % (w/w) to about 30 % (w/w), from about 10 % (w/w) to about 30 % (w/w), from about 15 % (w/w) to about 30 % (w/w), from about 20 % (w/w) to about 30 % (w/w), from about 25 % (w/w) to about 30 % (w/w), from about 5 % (w/w) to about 25 % (w/w), from about 10 % (w/w) to about 20 % (w/w), from about 5 % (w/w) to about 10 % (w/w), from 10 % (w/w) to about 15 % (w/w), from about 15 % (w/w) to about 20 % (w/w), or from about 20 % (w/w) to about 25 % (w/w)). In some cases, a hydrogel composition provided herein includes about 20% (w/w) tantalum particles such as tantalum microparticles.

In some embodiments, the hydrogels of the present disclosure comprise from about 10 % (w/w) to about 30 % (w/w) of tantalum particles, including about 15%, about 20 %, and about 25 %, and all values and ranges thereof. In some embodiments, the hydrogels of the present disclosure comprise from about 15 % (w/w) to about 25 % (w/w) of tantalum particles. In some embodiments, the hydrogels of the present disclosure comprise about 20 % (w/w) of tantalum particles.

A hydrogel composition provided herein can be visualized (e.g., within a mammal) using any appropriate method. For example, imaging techniques such as ultrasound, computed tomography, magnetic resonance imaging, and/or fluoroscopy can be used to visualize a hydrogel composition provided herein.

In some cases, a hydrogel composition provided herein can include 18% (w/v) gelatin, 9% (w/v) silicate nanoplatelets, and 20% (w/w) tantalum microparticles. In some embodiments, the present disclosure provides a hydrogel comprising gelatin, silicate nanoplatelets and tantalum microparticles. In some embodiments, the present disclosure provides a hydrogel consisting essentially of gelatin, silicate nanoplatelets, tantalum microparticles and water (e.g., deionized water).

In some embodiments, the hydrogels of the present disclosure comprise:
about 15 % to about 25% of tantalum particles (w/w);
about 2.5 % to about 6% of silicate nanoplatelets (w/w);
about 0.2 % to about 1.5 % of gelatin (w/w); and
water.

In some embodiments, the hydrogels of the present disclosure comprise:
about 17.5 % to about 22.5% of tantalum particles (w/w);
about 3.5 % to about 6% of silicate nanoplatelets (w/w);
about 0.6 % to about 1.0 % of gelatin (w/w); and
water.

In some embodiments, the hydrogels of the present disclosure comprise:
about 20% of tantalum particles (w/w);
about 4.75 % of silicate nanoplatelets (w/w);
about 0.8 % of gelatin (w/w); and
water.

In some embodiments, the hydrogels of the present disclosure comprise:
about 15 % to about 25% of tantalum particles (w/w);
about 2.0 % to about 5% of silicate nanoplatelets (w/w);
about 0.4 % to about 0.8 % of gelatin (w/w); and
water.

In some embodiments, the hydrogels of the present disclosure comprise:
about 20 % of tantalum particles (w/w);
about 4 % of silicate nanoplatelets (w/w);
about 0.7 % of gelatin (w/w); and
water.

In some embodiments, the hydrogels of the present disclosure comprise:
about 17.5 % to about 22.5% of tantalum particles (w/w);
about 2.5 % to about 4.5% of silicate nanoplatelets (w/w);
about 0.5 % to about 0.7 % of gelatin (w/w); and
water.

In some embodiments, the hydrogels of the present disclosure comprise:
about 20 % of tantalum particles (w/w);
about 3.5 % of silicate nanoplatelets (w/w);
about 0.6 % of gelatin (w/w); and
water.

In some cases, a hydrogel composition provided herein can be biodegradable. For example, a volume of a hydrogel composition delivered to a blood vessel within a mammal (e.g., a human) can decrease over time. In some cases, a volume of a hydrogel composition delivered to a blood vessel within a mammal (e.g., a human) can decrease by at least about 25% (e.g., at least about 30%, at least about 40%, at least about 50%, at least about 60%, at least about 70%, or at least about 75%) over time. In some cases, a volume of a hydrogel composition delivered to a blood vessel within a mammal (e.g., a human) can decrease for about 28 days following delivery. In some cases, a volume of a hydrogel composition delivered to a blood vessel within a mammal (e.g., a human) can decrease by about 75% for about 28 days following delivery.

In some cases, a hydrogel composition provided herein can be a shear-thinning hydrogel composition. For example, a viscosity of a hydrogel composition provided herein can decrease under a shear rate of about 10⁻¹ 1/s.

In some cases, a hydrogel composition provided herein can have a displacement pressure that is higher than the mean pressure of a blood vessel (e.g., healthy blood vessel or a blood vessel that has been embolized with coils). For example, a hydrogel composition provided herein can have a displacement pressure of from about 15 kPa to about 45 kPa.

A hydrogel composition provided herein can be made using any appropriate method. In some cases, gelatin and one or more nanosilicates can be mixed first, and then one or more radiopaque contrast agents can be added. For example, centrifugal mixing, manual mixing, high shear dispersing, vacuum mixing, vortexing, and/or syringe mixing can be used for mixing (e.g., homogenous mixing) of gelatin, one or more nanosilicates, and one or more radiopaque contrast agents to make a hydrogel composition provided herein. In some cases, a hydrogel composition can be made as described in Example 1.

Optionally, the hydrogel compositions of the invention can include one or more bioactive agents such as an embolic agent, an anti-inflammatory agent, an agent that modulates coagulation, an antibiotic agent, a chemotherapeutic agent or the like. Hydrogel compositions of the invention can be formulated for use as carriers or scaffolds of bioactive agents such as drugs, cells, proteins, and bioactive molecules (e.g., antibodies and enzymes). As carriers, such compositions can incorporate the agents and deliver them to a desired site in the body for the treatments of a variety of pathological conditions. Illustrative embolic agents include, for example, stainless steel coils, absorbable gelatin pledgets and powders, polyvinyl alcohol foams, ethanol, glues and the like. Illustrative hemostatic agents include, for example, Celox, QuikClot and Hemcon. Certain illustrative materials and methods that can be adapted for use in such embodiments of the invention are found, for example in Hydrogels: Design, Synthesis and Application in Drug Delivery and Regenerative Medicine 1st Edition, Singh, Laverty and Donnelly Eds; and Hydrogels in Biology and Medicine (Polymer Science and Technology) UK ed. Edition by J. Michalek et al. In addition, as scaffolds, compositions of the invention can provide a flexible dwelling space for cells and other agents for use in tissue repair and the regeneration of desired tissues (e.g. for cartilage, bone, retina, brain, and, neural tissue repair, vascular regeneration, wound healing and the like).

In some cases, a hydrogel composition provided herein can be sterilized. Any appropriate method can be used to sterilize a hydrogel composition provided herein. For example, irradiation (e.g., ionizing irradiation), gamma irradiation, electron beam irradiation, gas (e.g., ethylene oxide) based irradiation, and/or x-ray irradiation can be used to sterilize a hydrogel composition provided herein. In some cases, a hydrogel composition can be sterilized as described in Example 1.

Also provided herein are methods for using one or more hydrogel compositions provided herein. The hydrogel compositions provided herein can be used for embolization of one or more blood vessels within a mammal (e.g., a human). For example, one or more hydrogel compositions provided herein can be delivered to one or more blood vessels within a mammal for embolization of the blood vessel(s). In some cases, one or more hydrogel compositions provided herein can be used for embolization without fragmentation of the delivered hydrogel compositions. In some cases, one or more hydrogel compositions provided herein can be used for embolization without migration of the hydrogel compositions. In some cases, one or more hydrogel compositions provided herein can be used for embolization having a recanalization rate of less than about 35% (e.g., less than about 30%, less than about 25%, less than about 20%, less than about 15%, or less than about 10%).

In some cases, one or more hydrogel compositions provided herein can be delivered to one or more blood vessels within a mammal (e.g., a human) to reduce or eliminate blood flow within the blood vessel(s). For example, one or more hydrogel compositions provided herein can be delivered to one or more blood vessels within a mammal (e.g., a human) to reduce blood flow within the blood vessel(s) by for example, 10, 20, 30, 40, 50, 60, 70, 80, 90, 95, or more percent.

In some cases, one or more hydrogel compositions provided herein can be delivered to one or more blood vessels within a mammal (e.g., a human) to induce clotting within the blood vessel(s). For example, one or more hydrogel compositions provided herein can be delivered to one or more blood vessels within a mammal (e.g., a human) to induce clotting within the blood vessel(s) in less than about 20 minutes (e.g., less than about 15 minutes, less than about 12 minutes, less than about 10 minutes, less than about 8 minutes, or less than about 3 minutes). For example, one or more hydrogel compositions provided herein can be delivered to one or more blood vessels within a mammal (e.g., a human) to induce formation of a blood clot within the blood vessel(s) that has a mass or size (e.g., a volume) of from about 0.5 cubic centimeters (cm³) to about 5 cm³ (e.g., from about 0.5 cm³ to about 4.5 cm³, from about 0.5 cm³ to about 4 cm³, from about 0.5 cm³ to about 3.5 cm³, from about 0.5 cm³ to about 3 cm³, from about 0.5 cm³ to about 2.5 cm³, from about 0.5 cm³ to about 2 cm³, from about 0.5 cm³ to about 1.5 cm³, from about 0.5 cm³ to about 1 cm³, from about 1 cm³ to about 5 cm³, from about 1.5 cm³ to about 5 cm³, from about 2 cm³ to about 5 cm³, from about 2.5 cm³ to about 5 cm³, from about 3 cm³ to about 5 cm³, from about 3.5 cm³ to about 5 cm³, from about 4 cm³ to about 5 cm³, from about 4.5 cm³ to about 5 cm³, from about 1 cm³ to about 4.5 cm³, from about 1.5 cm³ to about 4 cm³, from about 2 cm³ to about 3.5 cm³, from about 2.5 cm³ to about 3 cm³, from about 0.5 cm³ to about 1.5 cm³, from about 1.5 cm³ to about 2.5 cm³, from about 2.5 cm³ to about 3.5 cm³, or from about 3.5 cm³ to about 4.5 cm³).

In some cases, one or more hydrogel compositions provided herein can be delivered to one or more blood vessels within a mammal (e.g., a human) having a bleeding disorder to treat the mammal. For example, a hydrogel composition provided herein can be delivered to one or more blood vessels feeding one or more tumors within the mammal to reduce or eliminate blood flow associated with the bleeding disorder. Examples of bleeding disorders that can be treated as described herein include, without limitation, hemorrhage (e.g., non-traumatic hemorrhage and traumatic hemorrhage), saccular aneurysms, vascular malformations, endoleaks, gastroesophageal varices (e.g., bleeding gastroesophageal varices), and arteriovenous fistulas.

In some cases, one or more hydrogel compositions provided herein can be delivered to one or more blood vessels within a mammal (e.g., a human) having one or more tumors to treat the mammal. For example, a hydrogel composition provided herein can be delivered to one or more blood vessels feeding one or more tumors within the mammal to reduce or eliminate blood flow to the tumor(s). In some cases, a tumor can be a malignant tumor. In some cases, a tumor can be a benign tumor. Examples of tumors that can be treated as described herein include, without limitation, hepatic tumors, uterine fibroids, prostate tumors, renal tumors, and cerebral tumors. For example, one or more hydrogel compositions provided herein can be delivered to one or more blood vessels feeding one or more tumors within a mammal (e.g., a human) to reduce the size (e.g., volume) of the tumor(s) by for example, 10, 20, 30, 40, 50, 60, 70, 80, 90, 95, or more percent.

In some cases, when one or more hydrogel compositions provided herein are delivered to one or more blood vessels within a mammal (e.g., a human), the mammal can experience minimal or no complications associated with embolization. Examples of complications associated with embolization include, without limitation, vasospasm, thrombosis, dissections, rupture, necrosis, bleeding at the puncture site, and hematoma at the puncture site.

One or more hydrogel compositions provided herein can be delivered to one or more blood vessels within any type of mammal. In some cases, a mammal (e.g., a human) can be anticoagulated (e.g., can be taking one or more anticoagulants). In some cases, a mammal (e.g., a human) can be coagulopathic (e.g., can have a bleeding disorder in which the mammal's blood's ability to coagulate is impaired). Examples of mammals that can have one or more hydrogel compositions provided herein delivered to one or more blood vessels within the mammal include, without limitation, humans, non-human primates such as monkeys, dogs, cats, horses, cows, pigs, sheep, mice, rats, and rabbits.

One or more hydrogel compositions provided herein can be delivered to any type of blood vessel within a mammal (e.g., a human). In some cases, a blood vessel can be a diseased blood vessel. In some cases, a blood vessel can be an injured blood vessel. Examples of types of blood vessels into which a hydrogel composition provided herein can be delivered include, without limitation, arteries, veins, and capillaries. When one or more hydrogel compositions provided herein are delivered to an artery, the artery can be any artery within a mammal (e.g., a human) such as a renal artery, an iliac artery, a gastric artery, a prostate artery, or a mesenteric artery. When one or more hydrogel compositions provided herein are delivered to a vein, the vein can be any vein within a mammal (e.g., a human) such as a portal vein, and a pelvic vein.

In some cases, one or more hydrogel compositions provided herein can be delivered to the lymphatic system within a mammal (e.g., a human).

In some cases, one or more hydrogel compositions provided herein can be delivered to the site of an endoleak (e.g., an endoleak of a vascular graft following repair).

One or more hydrogel compositions provided herein can be delivered to any size blood vessel within a mammal (e.g., a human). In some cases, a blood vessel can have a diameter (e.g., a luminal diameter) of from about 50 microns to about 10,000 microns (1 cm) (e.g., about 50 microns to about 5,000 microns, about 50 microns to about 1,500 microns, about 50 microns to about 1,000 microns, about 50 microns to about 900 microns, about 50 microns to about 800 microns, about 50 microns to about 700 microns, about 50 microns to about 600 microns, about 50 microns to about 500 microns, about 50 microns to about 400 microns, about 50 microns to about 300 microns, about 50 microns to about 200 microns, about 50 microns to about 100 microns, about 100 microns to about 10,000 microns, about 200 microns to about 10,000 microns, about 300 microns to about 10,000 microns, about 400 microns to about 10,000 microns, about 500 microns to about 10,000 microns, about 600 microns to about 10,000 microns, about 700 microns to about 10,000 microns, about 800 microns to about 10,000 microns, about 900 microns to about 10,000 microns, about 1,000 microns to about 10,000 microns, about 5,000 microns to about 10,000 microns, about 100 microns to about 5,000 microns, about 200 microns to about 1,000 microns, about 300 microns to about 900 microns, about 400 microns to about 800 microns, about 500 microns to about 700 microns, about 100 microns to about 400 microns, about 200 microns to about 500 microns, about 300 microns to about 600 microns, about 400 microns to about 700 microns, about 500 microns to about 800 microns, about 600 microns to about 900 microns, or about 700 microns to about 1,000 microns).

When delivering one or more hydrogel compositions provided herein to one or more blood vessels within a mammal (e.g., a human), any appropriate method of delivery can be used. In some cases, one or more hydrogel compositions provided herein can be delivered to one or more blood vessels within a mammal (e.g., a human) by injection directly to a blood vessel (e.g., a blood vessel in need of embolization). In some cases, one or more hydrogel compositions provided herein can be delivered to one or more blood vessels within a mammal (e.g., a human) by catheter-directed delivery (e.g., via a catheter inserted into a blood vessel in need of embolization). When one or more hydrogel compositions provided herein are delivered to one or more blood vessels within a mammal (e.g., a human) by catheter-directed delivery any type of catheter can be used (e.g., a Bernstein catheter, a microcatheter, a Cobra catheter, a Fogarty balloon, and a ProGreat catheter). When one or more hydrogel compositions provided herein are delivered to one or more blood vessels within a mammal (e.g., a human) by catheter-directed delivery any size catheter can be used. For example, one or more hydrogel compositions provided herein can be delivered to one or more blood vessels within a mammal (e.g., a human) using a catheter having a size of from about 2.8 French to about 5 French.

One or more hydrogel compositions provided herein can be delivered to one or more blood vessels within a mammal (e.g., a human) at any delivery rate. For example, one or more hydrogel compositions provided herein can be delivered to one or more blood vessels within a mammal (e.g., a human) at a rate of from about 1 mL/minute to about 2 mL/minute.

Any amount of one or more hydrogel compositions provided herein can be delivered to one or more blood vessels within a mammal (e.g., a human). For example, from about 2 mL to about 5 mL (e.g., from about 2 mL to about 4 mL, from about 2 mL to about 3 mL, from about 3 mL to about 5 mL, from about 4 mL to about 5 mL, from about 2 mL to about 3 mL, or from about 3 mL to about 4 mL) of one or more hydrogel compositions provided herein can be delivered to one or more blood vessels within a mammal (e.g., a human).

In some cases, after one or more hydrogel compositions provided herein are used for embolization of one or more blood vessels within a mammal (e.g., a human), the hydrogel composition(s) can be retrieved from the blood vessel(s). For example, after one or more hydrogel compositions provided herein are delivered to one or more blood vessels within a mammal for embolization of the blood vessel(s), the hydrogel composition can be retrieved to increase (e.g., restore) blood flow through the blood vessel(s). Any appropriate method can be used to retrieve one or more hydrogel compositions provided herein from one or move blood vessels within a mammal (e.g., a human). For example, aspiration catheters (e.g., aspiration through the delivery catheter), and surgical removal can be used to retrieve one or more hydrogel compositions provided herein from one or move blood vessels within a mammal (e.g., a human).

In some cases, one or more hydrogel compositions provided herein can be delivered to one or more blood vessels within a mammal (e.g., a human) as the sole active agent used for embolization.

In some cases, one or more hydrogel compositions provided herein can be delivered to one or more blood vessels within a mammal (e.g., a human) in combination with one or more additional agents used for embolization. For example, one or more hydrogel compositions provided herein can be delivered to one or more blood vessels within a mammal (e.g., a human) in combination with solid embolic materials (e.g., a coils, particles, foam, a plug, microspheres, and/or beads), liquid embolic materials (e.g., butyl cyanoacrylate (n-BCA), and Onyx^{®}).

In cases where one or more hydrogel compositions provided herein are used in combination with additional agents used for embolization, the one or more additional agents can be administered at the same time (e.g., in the same composition or in separate compositions) or independently. For example, one or more hydrogel compositions provided herein can be administered first, and the one or more additional agents administered second, or vice versa.

The invention will be further described in the following examples, which do not limit the scope of the invention described in the claims.

### EXAMPLES

In the following examples only the Ta-GEM compositions are according to the claimed invention. The remaining GEM compositions, i.e. compositions including gelatin and silicate nanoplatelets but without radiopaque contrast agent or with iohexol instead of tantalum particles as the contrast agent, are to be considered as reference examples

### Example 1: Catheter-Directed Injectable Biomaterial for Rapid Arterial Embolization

### Assessing GEM performance in a rat model of femoral artery occlusion

A major concern for gel-like embolic agents is the potential for fragmentation during delivery, which can lead to non-target distal embolization and unintended tissue ischemia. To evaluate the stability of GEM in a high velocity and highpressure vasculature, a rat model of femoral artery occlusion was created. In this model, arterial flow at the femoral artery (FA) is occluded with GEM; however, flow to the distal hindlimb is preserved by the collateral flow. Any breakdown of GEM will travel distally in the hindlimb of the rat and manifest as loss of perfusion to that digit or limb. Following the delivery of GEM directly into FA (Figure 1A,B), high-resolution laser speckle contrast imaging (LSCI) showed the absence of arterial flow (Figure 1C,D) confirming occlusion of the artery by GEM. To demonstrate flow to the hindlimb via collaterals, a 2 French microcatheter was delivered to the distal aorta from a carotid artery puncture, and digital subtraction angiography (DSA) using iohexol-based contrast agent was performed; this confirmed FA occlusion and patency of collateral vessels allowing blood flow to bypass the occluded segment of FA (Figure 1E). Following FA embolization, rats were then allowed to survive 0, 3, 7, or 21 days with a serial assessment of hindlimb perfusion using LSCI (Figure 1F,G). LSCI data showed that hindlimb perfusion to the digits was preserved, indicating that GEM in FA remained at the injected site without any fragmentation into the distal arterial system. Motor evaluation of the rats using the Tarlov scale also showed normal motor activity following FA embolization using GEM; this is consistent with angiography demonstrating persistent collateral perfusion to the hindlimb.

To assess durability and biodegradation of GEM in FA, quantitative micro-CT imaging and histology were performed at days 0, 3, 7, and 21. Axial images of FA at 10-micron resolution demonstrated uniform casting of the FA lumen, including smaller tortuous side branches such as the lateral circumflex artery (Figure 1H). The capability of GEM to enter smaller arterial branches is an important property as this can prevent retrograde bleeding in a hemorrhage scenario. Micro-CT images demonstrated persistent occlusion of the artery with progressive biodegradation (Figure 1H) and replacement of the biomaterial with fibrotic tissue, which was confirmed by histology (Figure 1I). Extensive 3D morphometric analysis of the micro-CT dataset over time (Figure 7A-D) supported structural changes associated with material remodeling inside the arterial lumen.

GEM embolization of the FA causes initial distension of the arterial lumen and thinning of the arterial wall; after 21 days, the arterial wall becomes thickened as the GEM biodegrades and remodels reducing arterial wall-tension and decreases arterial lumen area (Figure 7E-L). To assess the immune response to GEM embolization, myeloperoxidase (MPO) and CD68 immunostaining were performed (Figure 7G-H). The number of MPO stained cells within the arterial lumen peaked at day 3 post-embolization (Figure 7K), consistent with the acute inflammatory response to GEM as neutrophils infiltrated the arterial lumen from the periphery. In contrast, CD68 stained cells peaked at 21 days post-embolization (Figure 7L), suggesting that monocytes/macrophages play a role in the fibrotic remodeling process of the embolized arterial lumen.

To evaluate the efficacy of GEM embolization in a setting of anticoagulation, the rat model of FA occlusion was repeated in heparinized rats with activated clotting time (ACT) values over 300 seconds (Figure 8). Micro-CT images showed that heparin treatment did not change the morphometry of the GEM in FA (Figure 8D). Furthermore, LASCI images showed no difference in the distal perfusion of the hindlimbs between heparin treated and untreated rats (Figure 8E) and histology confirmed the presence of persistent casting of the FA lumen (Figure 8F,G). In this cohort of rats, GEM remained localized to the injected site of FA without migration, fragmentation or recanalization implying that GEM does not rely on thrombosis at the GEM-blood interface to prevent migration and non-target embolization.

To assess the systemic response to GEM embolization, a quantitative evaluation of 27 cytokines and chemokines was performed using the rat serum isolated at day 0, 3, 7, and 21 days. Results show that there was no significant increase in pro-inflammatory mediators such as TNF-α, IL-1β, IL-6, and MIP-1α in serum samples compared to baseline. In contrast, there was a significant increase in anti-inflammatory cytokines such as IL-1α, IL-10, and IL-4 peaking at day 7 and returning to baseline by 21 days. These suggest that intra-arterial GEM injection did not incite a systemic pro-inflammatory cytokine response, further supporting its biocompatibility and safety profile (Table 1).

**Table 1. Serum levels of cytokines and chemokines following femoral artery embolization in rats. Multiplex analysis of cytokines and chemokines measured in the rat serum at 3, 7, and 21 days following femoral artery embolization. Results show a significant increase in IL-1a, IL-10, and IL-4 levels that peaked at day 7 and then declined by Day 21 compared to baseline at Day-0 using analysis of variance. There was no change in the serum levels of pro-inflammatory cytokines compared to baseline control at Day-0. Results are mean ± SEM; unit is pg/ml, n=6 in each group (*P <0.05, **P <0.01; n=6).**

| | **Day 0** | **Day 3** | **Day 7** | **Day 21** |
|---|---|---|---|---|
| **G-CSF** | 3.1±2.2 | 9.9±6.8 | 14.7±10.5 | 2.7±3 |
| **Eotaxin** | 6.3±1 | 9.2±1.3 | 6.4±1.7 | 8.8±1 |
| **GM-CSF** | 22.7±4.3 | 19.8±9.4 | 28.7±4.7 | 27.9±2 |
| **IL-1α** | 5.1±1.2 | 18.5±3.1* | 27.3±8.4** | 8.6±2.1 |
| **Leptin** | 3754±339 | 1692±434* | 2742495 | 4106±961 |
| **MIP-1a** | 11±1 | 11.2±1 | 11.9±1.2 | 10.8±1.3 |
| **IL-4** | 9.8±2.4 | 11.6±3.2 | 18.6±2.2* | 12±2.8 |
| **IL-1β** | 26.1±4.2 | 36.5±6.5 | 39.9±5.1 | 30.9±9.2 |
| **IL-2** | 50.6±8.2 | 72.8±15.2 | 93.6±12.6 | 53.9±22.7 |
| **IL-6** | 488±148 | 566±122 | 820±327 | 413±60 |
| **IL-13** | 10.3±1.9 | 15.2±3.1 | 13.8±2.5 | 9.9±2.4 |
| **IL-10** | 12.9±2.8 | 17.8±3.4 | 31.6±3.7** | 19.1±3.6 |
| **IL-12** | 135±22 | 131±23 | 202±19* | 96±14 |
| **IL-5** | 50±3 | 58±6 | 49±3 | 43±5 |
| **IL-17** | 6.1±1.6 | 10.6±2.3 | 9.8±2.9 | 9.5±5.4 |
| **IL-18** | 113±14 | 136±18 | 145±24 | 102±16 |
| **MCP-1** | 926±81 | 1162±128 | 995±136 | 760±133 |
| **IP-10** | 145±19 | 180±32 | 159±29 | 141±20 |
| **VEGF** | 16.6±3 | 12.1±2.8 | 13±2.4 | 12.9±1.9 |
| **Fractalkine** | 36.8±3.1 | 37.8±2.9 | 39.3±1.6 | 38.6±3.5 |
| **LIX** | 1555±181 | 1218±168 | 1722±79 | 1639±204 |
| **MIP-2** | 48±2 | 29±6 | 20.2±4.7 | 20.2±4.2 |
| **TNFα** | 5.2±0.8 | 8.8±2.1 | 5.7±1.8 | 5.5±2.5 |
| **RANTES** | 792±181 | 500±144 | 634±110 | 779±232 |

### Enhancing GEM visibility to allow embolization in large animal models

Prior to testing GEM in a swine model of embolization, visualization of GEM during real-time X-ray fluoroscopy was optimized since this is critical to achieving targeted and controlled delivery. Clinically, metallic coils used today for embolization are visible during fluoroscopy; however, they produce significant artifacts with CT imaging, MRI, and US. The inability to assess the outcome of the intervention following embolization due to artifact is a major setback in clinical practice today. Here, it is shown that a formulation for GEM that would allow GEM and local anatomical visualization in all imaging modalities, including CT, MRI, US and fluoroscopy without significant artifact.

In rat experiments, GEM made with 10% iohexol was inadequate for visualization with fluoroscopy imaging, as shown in Figure 1E. To improve visualization of GEM, tantalum (Ta) particulates were used because Ta particulates are already in clinical use with various medical devices. To introduce sufficient radiopacity to allow visualization during fluoroscopy, Ta microparticles with an average size of 2 µm or Ta nanoparticles were mixed with GEM to form Ta-GEM (e.g., 2-30% w/w Ta-GEM) using a SpeedMixer. CT-phantom studies (Figure 2A,B) indicated that GEM mixed with 20% tantalum generated satisfactory visibility without any streak artifact. Fluoroscopy experiments showed that nano-Ta could not be uniformly distributed throughout GEM causing poor visibility; thus, subsequent experiments used the micron-size Ta particles in GEM (Figure 2C,D). To test the visibility of Ta-GEM *in vivo,* the hepatic vein in the pig liver was accessed percutaneously under ultrasound guidance using a 21G needle; in real-time, both US and fluoroscopic imaging were performed during Ta-GEM injection indicating sufficient visibility (Figure 2E,F). US imaging showed that GEM is echogenic, allowing direct visualization; furthermore, shear-wave US imaging indicated that the addition of Ta did not cause a significant increase in the stiffness of the embolic agent when compared to other samples with Ta (Figure 2G). Next, standard T1 and T2 based MRI of 20% Ta-GEM loaded into a syringe was performed. As expected, T1 sequences produced a dark image, and T2 based sequences produced a bright image; this was expected given MR properties of Ta and the hydrogel nature of GEM (Figure 2H-K). Analysis of GEM including micro-CT, CT, MRI, and scanning electron microscope imaging consistently demonstrated uniform dispersal of Ta in GEM representing effective speed mixing technique; this is important for providing a homogenous signal during imaging (Figure 2L,M).

### Evaluating Ta-GEM injectability and shear-thinning property

The capability to hand inject a syringe-filled Ta-GEM connected to a clinical catheter was a key practical requirement for the design of GEM. To determine whether Ta-GEM is suitable for transcatheter delivery, the injectability of GEM and Ta-GEM through 2.8 French and 5 French clinical catheters, respectively, was examined using compression testing (Figure 3A). In both systems, Ta-GEM demonstrated comparable injectability to that of GEM, suggesting that the addition of Ta particles did not impact injectability (Figure 3B-E). Next, the effect of interrupting delivery was investigated; no significant impact on the injection force was again observed (Figure 3F). The ability to perform intermittent delivery with real-time evaluation of embolization is an important property; today, this is not possible with current technologies.

In addition to injectability, the influence of Ta micro-particles on GEM's mechanical property was analyzed using rheological studies. The flow curves of GEM and Ta-GEM demonstrate similar shear thinning characteristics (Figure 3G). To assess the embolic strength of GEM and Ta-GEM, amplitude oscillation tests were performed to measure their storage modulus G' and loss modulus G". Figure 3H demonstrates that both GEM and Ta-GEM are viscoelastic, and they possess G' values that are approximately 10 times of G". Furthermore, no significant change in G' (measured at 0.1% strain) was observed in Ta-GEM compared to GEM (Figure 3I), suggesting that Ta had minimal impact on GEM's mechanical properties. These results suggested comparable embolic strength between GEM and Ta-GEM; this was further confirmed by *in vitro* embolization experiments (Figure 3J-L). The displacement pressure of GEM and Ta-GEM are similar (35.8±3.9 kPa and 33.2±2.6 kPa, respectively; p=0.5), but significantly higher than coils alone (9.9±1.5 kPa, p<0.0001). More importantly, when Ta-GEM is combined with coils, the displacement pressure reaches up to 234.5±21.4 kPa or nearly 15 times greater than the standard physiologic systolic pressures in humans. The additional strength from GEM combined with coils may be due to increased friction between Ta particles and coils.

### Effect of tantalum on GEM thrombogenicity

Enhanced thrombogenicity of Ta-GEM is a favorable trait in an embolic agent to achieve vascular occlusion. Blood coagulation in aliquots of citrated human blood mixed with Ta-GEM was compared to standard coils used in patients. Both coils, GEM and Ta-GEM demonstrated clot promoting properties when compared to blood alone; however, when the mass of the clot is measured, Ta-GEM demonstrated a significant rise in mass beginning at 3 minutes compared to GEM and blood alone (Figure 9A,B). These results suggest that Ta-GEM has enhanced clot formation properties, which may further stabilize the embolic material at the blood-GEM interface.

### GEM Sterilization

To sterilize Ta-GEM, a desktop X-ray irradiator (RS2000, RAD.SOURCE) was used. Following 11 cGy/minutes of ionizing radiation for a total dose of 12000 rads exposure to GEM, which is sufficient to eradicate bacteria and fungus, there was no detectable microbial growth in the irradiated samples confirming its sterility (Figure 9C). Furthermore, GEM prepared in non-sterile conditions on a lab benchtop also did not show any evidence for microbial growth, suggesting that the composition of GEM itself is anti-bacterial.

### Testing Ta-GEM in a swine model of embolization

The embolization efficacy of GEM was tested in the swine arterial system. Experiments were designed to evaluate the performance of GEM compared to coils in the embolization of first-order arteries in a state of extreme anticoagulation. It was also investigated whether coils that failed to achieve hemostasis could be subsequently rescued by GEM injection into the coil mass and whether GEM could be retrieved from the artery following injection. Today, no embolization coil can achieve hemostasis in a state of anticoagulation or coils designed to be removed after delivery.

### Iliac artery embolization in a swine model using GEM

Using clinical tools and imaging equipment, a combination of wire and a catheter was used to deliver the catheter tip to the distal abdominal aorta. Digital subtraction angiography (DSA) demonstrated the characteristic split of the aorta into the paired external and internal iliac arteries. Following injection of 20-30,000 Units of IV Heparin to achieve ACT >300 seconds, the iliac artery was catheterized; approximately 6-7 cm of the iliac artery was subsequently embolized with GEM through a 5 French Bernstein catheter or a 2.8 French microcatheter (Figure 4; real-time delivery of GEM *in vivo* is demonstrated in Figure 10A-C). DSA from the aortic bifurcation following embolization demonstrated immediate and complete focal occlusion of the target artery (Figure 4D, white arrow). In comparison, detachable, 0.46 mm or 0.89 mm (0.018 inch or 0.035 inch)-thick coils of various lengths were used to embolize the contralateral iliac artery; despite deploying approximately 50-80 cm of coils in a state of ACA >300, these coils did not achieve hemostasis for the duration of the experiment, which is consistent with clinical experience (Figure 4D,E; black arrow). In addition, on average, embolization time using GEM was 40X faster than coil embolization; P < 0.00001 (Figure 4D); this demonstrates the simplicity and practicality of GEM delivery, which is a unique aspect of GEM.

Next, it was tested whether coils that consistently failed to achieve occlusion and hemostasis, e.g., Figure 4D, could be rescued by injection of GEM. In 8 cases where coils failed in a state of anticoagulation, 2-3 mL of GEM injection to the proximal end of the coil mass enabled instant hemostasis and occlusion (Figure 4F). Micro-CT imaging of the embolized segments harvested following necropsy demonstrated uniform casting of the iliac arteries with GEM without any CT imaging artifact even at high-resolution imaging, allowing the ability to assess outcomes of the intervention (Figure 4G, arrowhead). However, in the adjacent arterial segment that was coil embolized, significant streak-artifact was noted precluding the assessment of the embolized and adjacent arteries (Figure 4G, white arrow). These streak artifacts are a common occurrence in patients who receive CT scans; they limit evaluation of the treatment zone and, in cerebral aneurysm cases, often leads to invasive angiography to evaluate for embolization efficacy.

Non-target embolization is always a concern in embolization procedures. As a safety mechanism, it was evaluated whether GEM could be retrieved in the event of an unintended or accidental non-target embolization. In 5 cases, the iliac artery was embolized using GEM; after up to 3 hours, GEM was retrieved in its entirety using the Penumbra Aspiration catheter system (Fig 4H-J; Figure 10E-H), a device FDA approved for stroke thrombectomy, restoring flow. These results indicate that GEM could be retrieved. These results also suggest that, for the first time, temporary embolization may be a possibility where GEM could be retrieved to restore normal flow, for example, in vascular trauma patients.

### Survival experiments in a swine model of iliac artery embolization using GEM

Long term outcomes of using GEM in the embolization of swine iliac arteries while receiving daily antiplatelet therapy for anticoagulation were investigated. Following up to 28 days of survival post-embolization, the animals underwent contrast-enhanced CT angiogram (CTA) and then were euthanized. All 16 animals demonstrated persistent embolization at each time point with no evidence for non-target embolization on CTA imaging (Figure 5). The embolized iliac arteries were occluded with GEM devoid of any flow to suggest recanalization; lower hindlimb perfusion from the aorta to the ankles via collaterals were also preserved (Figure 11A-C). There were no focal bright spots within the pelvic or hindlimb muscle compartments to suggest fragmentation and dislodgement of GEM (Figure 11B). A board-certified radiologist's general review of the pig CTA images revealed unremarkable findings (Figure 11D-L); for example, there was no evidence for lymphadenopathy, and the solid organs included the lungs, spleen, kidneys, and liver were normal. Subsequently, during necropsy, specimens from these organs were also collected for histologic evaluation; a review of these slides by a board-certified pathologist showed normal findings. During necropsy, the pelvic arteries were also dissected and immediately imaged using a high-resolution micro-CT scanner. Image analysis of micro-CT data compared to corresponding histology sections revealed progressive biodegradation of the GEM over the 4 weeks (Figure 5). Segmented and 3D reconstructed iliac arteries using the micro-CT dataset ranging from 700 Gb to 1 TB show progressive loss of GEM volume (Figure 5B, the black inside each artery represents GEM) and a decrease in the luminal diameter of the embolized arteries. After 4 weeks, micro-CT volumetric analysis revealed that approximately 75% of the GEM had biodegraded (Figure 5D). On Day 0, corresponding histology images showed uniform occlusion of the iliac artery with no significant tissue reaction and the absence of cellular infiltration (Figure 5A). However, over time, a concentric fibro-inflammatory reaction ensues in the arterial lumen rich in macrophages, myofibroblasts and fibrin resembling granulation tissue. The biomaterial becomes infiltrated with MPO positive granulocytes and scattered multinucleated giant cells as GEM gradually gets replaced with connective tissue. The majority of the granulocytes were within the lumen of the artery (Figure 5A and Figure 12) and both the internal and external elastic laminas showed evidence for disruption with breakdown of the elastic fibers of the intima and media layers and marked fibrosis of the media layer (Figure 5A, Figure 12C-E). With progressive fibrosis of the lumen, the luminal diameter was decreased at 4 weeks following an initial distention with GEM embolization (Figure 5, H&E and Trichrome stain). The medial thickness of the artery did not change over 4 weeks in contrast to the rat arterial wall.

To assess for systemic toxicity and inflammatory response to GEM, complete blood count (CBC), basic metabolic panel (BMP), liver function tests (LFT), and a cytokine array analysis were performed; samples before embolization were compared to blood samples obtained just prior to sacrifice. Blood tests before and after embolization including CBC, LFTs and BMP were unremarkable (P>0.05, Table 2); this is a significant result because it indicates that GEM does not consume platelets, activate acute phase reactants, increase white blood cell counts because of an infection, or injure solid organs. Cytokine array analysis of the blood before and after embolization demonstrated no signs of inflammation, with most factors reduced in post embolization samples (Table 3). These blood tests, cytokine analysis, histology, and cross-sectional imaging of the pigs further demonstrate that GEM is biocompatible and safe without evidence of an adverse systemic response.

**Table 2. Complete blood count and serum chemistry profile in pigs following arterial embolization with GEM. Table shows blood values measured at 0, 1, 2, or 4 weeks following internal iliac artery embolization. WBC, white blood cells; HTC, hematocrit; MCHC, mean corpuscular hemoglobin concentration; MCV, mean corpuscular volume; RDW, erythrocyte distribution width; HGB, hemoglobin; RBC, red blood cells; MPV, mean platelet volume: ALP, alkaline phosphatase, CRE, creatinine; ALT, alanine aminotransferase; BUN, blood urea nitrogen. All measured values remained within the normal limits for Yorkshire swine. Results are mean ± SEM, unit, picogram per mL, n=4 in each group (*P <0.05).**

| | **0 Week** | **1 Week** | **2 Week** | **4 Week** |
|---|---|---|---|---|
| **WBC** | 11.18 ± 1 | 15.5 ± 0.76 | 13 ± 1.67 | 24 ± 7 |
| **Lymphocyte** | 6.5 ± 0.5 | 9 ± 0.46 | 7.6 ± 0.58 | 15.5 ± 5.8 |
| **Monocyte** | 0.78 ± 0.1 | 1.15 ± 0.1 | 1 ± 0.08 | 1.97 ± 0.7 |
| **Granulocyte** | 4 ± 0.5 | 5.2 ± 0.3 | 4.5 ± 1 | 7.4 ± 1.3 |
| **Lym%** | 56 ± 6.9 | 59 ± 0.9 | 61 ± 3 | 59.3 ± 5.7 |
| **Mon%** | 6.0 ± 0.7 | 7 ± 0.1 | 6 ± 0.3 | 7.6 ± 0.4 |
| **Gra%** | 38 ± 6.3 | 34 ± 0.8 | 33 ± 3 | 33.1 ± 6.0 |
| **HCT** | 22.8 ± 0.6 | 26 ± 1.1 | 25 ± 2.2 | 27 ± 1.8 |
| **MCV** | 43.6 ± 1.1 | 45 ± 0.3 | 43 ± 1 | 44.8 ± 0.9 |
| **RDWa** | 26.7 ± 0.6 | 28 ± 0.4 | 25 ± 1 | 26.8 ± 0.5 |
| **RDW%** | 22 ± 0.9 | 22 ± 0.7 | 21 ± 1 | 21.5 ± 1.1 |
| **HGB** | 9 ± 0.2 | 10 ± 0.4 | 9.4 ± 0.8 | 9.8 ± 0.6 |
| **MCHC** | 38.4 ± 0.3 | 38 ± 0.3 | 3 9 ± 1 | 36.4 ± 0.2 |
| **MCH** | 16.8 ± 0.5 | 17 ± 0.2 | 17 ± 1 | 16.3 ± 0.3 |
| **RBC** | 5.2 ± 0.2 | 5.86 ± 0.3 | 5.82 ± 0.6 | 6.0 ± 0.3 |
| **Platelets** | 330 ± 57 | 371 ± 23 | 283 ± 79 | 319.0 ± 75 |
| **MPV** | 6.5 ± 0.2 | 6 ± 0.2 | 6 ± 0.1 | 6.5 ± 0.2 |
| **TP g/dl** | 5 ± 0.1 | *6.5 ± 0.1 | 6.4 ± 0.14 | 6.7 ± 0.2 |
| **ALP U/L** | 186 ± 7 | 165 + 16 | 156 ± 23 | 146 ± 9.0 |
| **GLU mg/dl** | 81±10.5 | 124 ± 6.6 | 112 ± 3.1 | 103 ± 7.0 |
| **ALT U/L** | 36 ± 1.7 | *89.5 ± 21 | 42 ± 1.2 | 40.3 ± 3 |
| **CRE mg/dl** | 1.34 ± 0.06 | 1.5 ± 0.2 | 1.67 ± 0.1 | *1.7 ± 0.2 |
| **BUN mg/dl** | 9 ± 0.9 | 14 ± 1.6 | 15.5 ± 1.22 | 21.8 ± 0.8 |

**Table 3. Serum levels of cytokines and chemokines following arterial embolization in pigs. Multiplex analysis of cytokines and chemokines at 0, 1, 2, or 4 weeks following internal iliac artery embolization. Results show no significant increase in all measured values. Results are mean ± SEM, unit, picogram per mL, n=4 in each group. GM-CSF, Granulocyte-macrophage colony-stimulating factor; IFNy, interferon-gamma; IL, interleukin; TNF, tumor necrosis factor.**

| | **0 Week** | **1 Week** | **2 Week** | **4 Week** |
|---|---|---|---|---|
| **GM-CSF** | 87 ±69 | 24±24 | 137±80 | 73±68 |
| **IFNy** | 14193±4335 | 2633±1244 | 9258±7924 | 7637±2928 |
| **IL-1α** | 57±19 | 12±5 | 60±41 | 26±12 |
| **IL-1β** | 537±186 | 130±60 | 597±392 | 205±116 |
| **IL-1rα** | *2390±956 | 130±60 | 713±217 | 216±44 |
| **IL-2** | 445±161 | 134±54 | 502±335 | 262±128 |
| **IL-4** | 2964±1308 | 326±168 | 4206±3433 | 2140±1032 |
| **IL-6** | 106±33 | 36±22 | 212±169 | 89±61 |
| **IL-8** | *355±118 | 79±26 | 298±166 | 88±26 |
| **IL-10** | 1311±475 | 219±53 | 1411±1065 | 668±394 |
| **II-12** | 932±106 | 888±56 | 1355±68 | 1406±369 |
| **IL-18** | 1678±546 | 609±119 | 2200±1326 | 911±435 |
| **TNFα** | 468±327 | 35±30 | 27±17 | 30±18 |

### Survival experiments in a swine model of renal artery embolization using GEM

Iliac artery embolization demonstrated that GEM is stable with permanent occlusion (e.g., arterial occlusion without any evidence of flow through on angiographic imaging indicating that the embolization was stable) over 28 days. However, recanalization of the embolized arterial segment could not be excluded with certainty since CT images distal to the embolized iliac artery revealed contrast enhancement; this could have resulted from extensive cross-pelvic collateral flow bypassing the embolized segment or from recanalization. To assess for possible recanalization of GEM over time, the main renal artery in 16 pigs was embolized. The kidney is comprised of end-organ arteries; successful embolization of the main renal artery would result in distal ischemia and atrophy of the kidney. However, any recanalization of the GEM would lead to the restoration of blood flow and reduced or absence of atrophy. Furthermore, because the renal artery is an end-artery, the smallest vessel that could be embolized with GEM was also determined and confirmed by histology.

Using a 5 French Cobra catheter, the main renal artery was catheterized, confirmed by DSA imaging (Figure 6A). Subsequently, 2-3 mL of GEM was injected leading to the instant casting of the artery and occlusion (Figure 6B). DSA imaging from the aorta using the Cobra catheter consistently showed complete occlusion of the renal artery. Just prior to necropsy, imaging using a clinical CT scanner was performed (Figure 6C-E); these revealed the absence of flow to the embolized kidney and progressive atrophy over the 28 day study period. By 4 weeks, the embolized kidney demonstrated a consistent, approximately 4-fold decrease in the kidney volume without any flow in the embolized renal artery or any distal arterial segment (Figure 6F-K); these data suggest that there was no evidence for recanalization. Next, it was assessed for any evidence for fragmentation and non-target embolization; because the kidney is an end-organ artery, the 5-8 micron capillaries would capture any dislodged GEM and would be visible by micro-CT, near-infrared Spectrum imaging and by histology (Figure 13). This extensive analysis revealed that the cortex of the kidney did not contain any evidence for GEM. On further analysis, to determine the smallest artery that GEM could flow into, a 4 French Fogarty balloon was inflated in the main renal artery to fix its position followed by GEM injection through this Fogarty catheter; injection was stopped when the Fogarty balloon demonstrated proximal displacement (Figure 13). Subsequent high-resolution micro-CT imaging revealed that the smallest artery GEM could be detected in measured approximately 320 microns (Figure 13D-F). Furthermore, evaluation of the chest by a board-certified radiologist using axial and reformatted coronal images revealed no evidence of focal hyperdensity or atelectasis to suggest migration of the embolic material from the renal arterioles to the capillaries (Figure 11E-F). Axial, coronal and sagittal images of the brain and extremity digits were also negative for non-target embolization and any evidence for ischemic changes or microvascular infarcts (Figure 11).

### Gelatin based Gelfoam embolization in swine renal arteries

The embolization performance of GEM was compared to gelfoam, which is clinically used today for hemorrhage control. Similar to the gelatin used in GEM, gelfoam is also comprised of porcine gelatin. Pre-cut gelfoam (EmboCube, Meritt Medical) was mixed with 50% v/v saline and 50% iohexol to create a slurry. These were subsequently used to embolize the renal artery to immediate occlusion (Figure 6L-M). Following two weeks of survival, animals were imaged using a clinical CT scanner; these images demonstrated contrast enhancement of the kidney resulting from recanalization and persistent blood flow within the renal artery (Figure 6N). This was confirmed by the absence of gelfoam inside the renal artery at necropsy and histology. These data suggest that GEM outperforms coils and gelfoam, the current clinical tools used today for embolization.

### Experimental Section

### GEM synthesis and sterilization

Iohexol-free GEM was prepared by mixing 18% (w/v) gelatin (Type A, Sigma Aldrich, St. Louis, MO, USA), 9% (w/v) silicate nanoplatelets (Laponite XLG, BYK USA Inc., Rochester Hills, MI, USA) and ultrapure water at a weight ratio of 1:6:5, according to a as described elsewhere (see, e.g., Avery et al., Sci. Transl. Med., 8:365ra156 (2016); and Gaharwar et al., ACS Nano., 8:9833 (2014)). To introduce radiopacity, GEM was mixed with iohexol or tantalum particles. Iohexol solution (Omnipaque 350 mgI/mL, GE HealthCare, MA) was mixed into GEM to achieve a 10% w/w final concentration. Tantalum microparticles (Ta) with an average size of 2 µm (Alfa Aesar, Haverhill, MA, USA) or tantalum nanoparticles <25 nm particle size (Sigma-Aldrich, St. Louis, MO) were mixed with GEM at various w/w levels to form GEM-Ta hydrogel (e.g., 20% w/w Ta GEM). The homogenous mixing of all GEM formulations was achieved by using a SpeedMixer (FlackTek Inc., Landrum, SC). To sterilize GEM, a RS2000 irradiator system (RAD.SOURCE) was used to expose GEM loaded syringes to 160 kV, 25 mA of ionizing irradiation dose equivalent to 11 cGy/minute for a total of 12000 rads based on an established protocol to eradicate bacteria and fungus. To confirm sterility, standard microbial growth assay using LB agar plates or LB broth with and without 100 mg/mL ampicillin was performed. LB broth tubes containing 10⁷-10⁸ CFU of chemically competent *E. coli* bacteria were used as a positive control. LB broth tubes which had 0.1 mL PBS alone served as a negative control. Quadruplicate tubes from GEM batches and each control were prepared and incubated on a shaking platform inside a 37° C incubator for up to 7 days. The analysis was performed as described in Avery et al., Sci. Transl. Med., 8:365ra156 (2016).

### Rheological Testing

Flow curves and amplitude sweeps were performed as described elsewhere (see, e.g., Avery et al., Sci. Transl. Med., 8:365ra156 (2016)). The rheological evaluation of GEM was performed using an Anton Paar MCR 302 rheometer (Anton Paar USA Inc., Torrance, CA, USA). A sandblasted 25 mm diameter aluminum upper plate and an aluminum lower plate, with a 500 µm gap in between, were used for all measurements. Flow curves and amplitude sweeps (at 10 rad/s) were obtained at 25 °C and 37 °C. For tests at 37 °C, the solvent trap was used, and the edge of the solvent trap was filled with water to provide a humidified environment. Data were acquired at least in triplicates for each experiment.

### Analysis of GEM Injectability

Injectability was examined using a mechanical tester equipped with a 100 N load cell (Instron, Norwood, MA) according to the previously established protocols. Briefly, GEM was loaded into 1mL or 3mL luer-lock syringes (Medallion, Merit Medical, South Jordan, UT) and injected through a 110 cm 2.8 French ProGreat catheter (Terumo Medical Corporation, Somerset, NJ, USA) or a 100 centimeter 5F Bernstein catheter (Cook Medical Inc, Bloomington, IN), respectively. GEM containing syringes was inserted inside of a custom-designed 3D printed holder, and the plunger was placed against the load cell plate; the compression force was applied at either 2 mL/minute or 1 mL/minute rate for 5F and 2.8F respectively. To simulate injection during clinical practice, interrupted compression force was also applied for 10 seconds, followed by a 5 second pause; this cycle was repeated 8 times through a 5 French catheter. The generated break loose and injection forces overtime was acquired and plotted using Bluehill version-3 software (Instron, Norwood, MA, US). Injectability testing was repeated at least five times for each condition.

### Assessing the effect of GEM on Blood Clotting Time

Clotting time and thrombus weight were quantified as described elsewhere (see, e.g., Avery et al., Sci. Transl. Med., 8:365ra156 (2016); and Gaharwar et al., ACS Nano., 8:9833 (2014)). Briefly, 0.5 grams of GEM aliquots were weighed into 2 mL microtubes. GEMs were centrifuged at 1000 RPM to standardize the blood interaction surface. Uncoagulated citrated blood was reactivated by adding 10% (v/v) 0.1 M CaCl₂. 100 µL of activated blood was added to each GEM sample and allowed to react for 3, 8, 10, 12, 15, or 20 minutes. At each time point, clotting was stopped by the addition of 200 µL of 0.109 M sodium citrate solution. Residual liquid was removed, isolating the clotted blood. The clotted blood was weighed in each test tube to determine the mass.

### Rat model of femoral artery occlusion

All *in vivo* studies were approved by the institutional animal care and use committee (IACUC) and conducted in accordance with federal and institutional guidelines. The right femoral artery was dissected between the proximal profunda and the superficial caudal epigastric artery away from the femoral vein and nerve. Two 4-0 silk ligatures were looped around the distal and proximal ends to manipulate and stabilize the artery segment during the injection. A micro-vessel clamp was placed distal to the profunda branch. Following GEM injection using a 27-gauge needle, rats were randomly divided into four groups and survived for 0, 3, 7, or 21 days (n=6 in each group). Rats in the 0-day time-point were euthanized 1 hour following GEM embolization; these served as the control group. Each rat was serially imaged using laser speckle contrast imaging (LSCI) to quantify hind limb microperfusion, and motor function was assessed using the modified Tarlov scale. Imaging was performed by placing anesthetized rats in a prone position on a warming platform to maintain core temperature at 37°C. Following 5 minutes of stabilization, the laser was positioned over the rat at a set distance of 20 centimeters, and perfusion imaging was initiated. Data were acquired at baseline, immediately after injection, and at day 1 post-surgery, and subsequently once a week afterward. Perfusion data were calculated as a ratio of perfusion in the GEM injected hind limb paw to the contralateral non-injected hind limb paw and expressed as a percent of baseline.

### Femoral artery occlusion in anticoagulated rats

Six rats received heparin before GEM injection into the femoral artery as described above and continued to receive anticoagulation therapy for 3 days post-GEM injection. Activated clotting time (ACT) was documented using iSTAT analyzer (Abbot, Princeton, NJ) in a 0.25 mL blood aliquot withdrawn from the caudal superficial epigastric vein before and after heparin administration. Once baseline ACT value was recorded, 1200 IU/kg heparin diluted in 250 µL normal saline was intravenously infused into the femoral vein branch. Subsequently, these rats received 150 IU/kg heparin subcutaneously twice a day for 3 days following GEM injection. A parallel group of 6 rats received a similar volume of saline instead of heparin serving as the control group.

### Percutaneous endovascular embolization in pigs

This study was approved by the institutional animal care and use committee (IACUC). Yorkshire pigs (S&S Farms, Brentwoods, CA) weighing 48 to 55 kg were acclimatized for at least 4 days. Anesthesia was induced using intramuscular injection of 5 mg/kg tiletamine-zolazepam (Telazol, Zoetis), 2 mg/mL xylazine and 0.02 mg/kg g lycopyrrolate. Pigs were then placed in a supine position and intubated on an X-ray compatible operating table (Pannomed Aeron, DRE, KY). Following intubation, anesthesia was maintained with inhalation of 1.5-3% isoflurane. During the procedure, electrocardiogram, transcutaneous oxyhemoglobin saturation (SpO₂), end-tidal CO₂ concentration, inspired oxygen fraction, and core temperature was monitored. Percutaneous access to the carotid artery was obtained using ultrasound guidance (ACUSON S2000, Siemens) and fluoroscopy (OEC9800 plus C-Arm, GE Healthcare Systems, Chicago, IL). Access needle and wire were exchanged for a 5-French Bernstein catheter (Cook Medical). Over a GT-glidewire (Terumo Medical), the tip of the catheter was advanced to the renal or the iliac artery using contrast-enhanced fluoroscopy (350 mgI/mL Omnipaque, GE HealthCare, MA). Pigs received 10,000-30000 IU of heparin IV, and ACT levels were documented using iSTAT analyzer (Abbot Laboratories Princeton, NJ). GEM or EmboCube (Merit Medical) was delivered to the 1^{st} order arterial branches iliac or renal artery using a catheter under real-time fluoroscopic guidance. Syringes with the embolic agent were connected directly to the catheter using the l uer-lock. In a subset, iliac arteries received metallic coils, including those from Medtronic, Terumo, Cook Medical, and Boston Scientific. The time to deployment of GEM or coils was recorded for each procedure. Following embolization, angiography was repeated multiple times to assess vessel patency. Pigs were either euthanized 1-hour post-embolization (non-survival group) or at 1, 2, or 4 weeks post-embolization (survival group). Prior to euthanasia, blood samples were obtained for analysis, and whole animal CTA imaging was performed. At necropsy, vascular tissues containing GEM or coils were explanted for µCT imaging and histopathology evaluation. Tissues from the liver, spleen, heart, and normal kidneys were also obtained for histology review to assess for potential toxicity.

### CT, US, MRI and Fluoroscopy Acquisition Methods

CT acquisition was performed on a dual-source scanner (Siemens Force, Siemens Healthineers, Erlangen, Germany). The spiral scan was performed at 150 kVp and 80 kVp energy level, respectively with a 0.6 mm detector size configuration. The MRI acquisition was performed on a 3.0 T scanner (Siemens Skyra, Erlangen, Germany). The 20-channel head coil with the main body transmit coil was used to acquire the data. MRI acquisition consisted of axial single-shot T2, axial dual-echo in-phase and out-of-phase, T1-weighted imaging before, and balanced steady-state free precession. The Fluoroscopy acquisition was performed on a mobile C-arm (OEC 9800 Plus, GE Healthcare, Illinois, USA).

### MicroCT imaging and structural analysis

Explanted embolized vessels were fixed with 10% formalin and incubated in 70% ethanol for 24 hours. MicroCT imaging was performed using SkyScan 1276 (Bruker, Kontich, Belgium) at 33 kV, 223 µA for rat vessels and 45 kV and 200 µA for pig arteries at a pixel size of 10 µm. Data analysis was performed using NRecon, CTvox, Data Viewer and CTAn software (Bruker, Kontich, Belgium). To document structural changes in the injected GEM, a quantitative morphometric analysis was performed to calculate surface to volume ratio, surface convexity index, fractal dimension, and structure model index. To calculate GEM volume inside the embolized pig vessels, Mimics software (Materialise, Leuven, Belgium) was used to segment GEM and the vessel lumen.

### Histopathology and immunohistochemistry

Paraffin-embedded sections were stained with H&E, Mason's trichrome or EVG elastin stain, and immunostaining for myeloperoxidase (MPO; ab208670, Abcam) or CD68 (ab125212, Abcam) was performed as described elsewhere (see, e.g., Avery et al., Sci. Transl. Med., 8:365ra156 (2016)). Morphometric analysis was performed using image analysis software (Celleste 4.1, Thermo Fisher Scientific). A blinded observer counted MPO or CD68 positive cells.

### Multiplex analyses of serum

Selected biomarkers, including cytokines, chemokines, and growth factors were measured in serum samples obtained from aliquots of blood samples from rats at 0, 3, 7 and 21 days and from pigs at 0, 1, 2, and 4 weeks post-embolization. The rat's serum samples were analyzed using the cytokine/chemokine array 27-plex, while the pig samples we analyzed using the porcine cytokine/chemokine array 13-plex (Eve Technologies, Calgary, CA). The analyte concentrations are expressed in picogram per mL.

### Statistical analysis

Analyses between groups at different time points were performed using analysis of variance (ANOVA), with post hoc multiple comparison procedures. A comparison between the two groups was calculated using the student t-test. Statistical significance in inter-group differences was assessed at the 95% confidence level (p<0.05). Data are presented as mean ± standard errors of the mean (SEM). All comparisons we obtained using GraphPad Prism 7 (GraphPad Software, Inc., La Jolla, CA).

### OTHER EMBODIMENTS

It is to be understood that while the invention has been described in conjunction with the detailed description thereof, the foregoing description is intended to illustrate and not limit the scope of the invention, which is defined by the scope of the appended claims.

## Claims

1. A hydrogel composition comprising:
(a) gelatin;
(b) a nanosilicate; and
(c) a radiopaque contrast agent;
wherein said nanosilicate is a silicate nanoplatelet, and wherein said radiopaque contrast agent comprises tantalum particles.

2. The hydrogel composition of claim 1, wherein said hydrogel composition comprises from about 0.1 % (w/v) to about 20 % (w/v) of said gelatin.

3. The hydrogel composition of claim 2, wherein said hydrogel composition comprises 18% (w/v) of said gelatin.

4. The hydrogel composition of any one of claims 2-3 wherein said gelatin is a Type A gelatin.

5. The hydrogel composition of claim 1, wherein said hydrogel composition comprises from about 3 % (w/v) to about 9 % (w/v) of said nanosilicate.

6. The hydrogel composition of claim 5, wherein said hydrogel composition comprises about 9% (w/v) of said nanosilicate.

7. The hydrogel composition of claim 1, wherein said hydrogel composition comprises from about 2 % (w/w) to about 30 % (w/w) of said radiopaque contrast agent.

8. The hydrogel composition of claim 1, wherein said hydrogel composition comprises about 20% (w/w) of said radiopaque contrast agent.

9. The hydrogel composition of claim 1, comprising
about 15 % to about 25% of tantalum particles (w/w);
about 2.5 % to about 6% of silicate nanoplatelets (w/w);
about 0.2 % to about 1.5 % of gelatin (w/w); and
water.

10. The hydrogel composition of claim 1, comprising
about 15 % to about 25% of tantalum particles (w/w);
about 2.0 % to about 5% of silicate nanoplatelets (w/w);
about 0.4 % to about 0.8 % of gelatin (w/w); and
water.

11. A hydrogel composition as defined in any one of claims 1-10 for use in a method for embolization of a blood vessel within a mammal, said method comprising delivering the hydrogel composition to said blood vessel.

12. A hydrogel composition as defined in any one of claims 1-10 for use in a method for inducing clotting in a blood vessel within a mammal, said method comprising delivering the hydrogel composition to said blood vessel.

13. A hydrogel composition as defined in any one of claims 1-10 for use in a method for treating a mammal having a bleeding disorder, said method comprising delivering the hydrogel composition to a blood vessel within said mammal.

14. A hydrogel composition as defined in any one of claims 1-10 for use in a method for treating a mammal having a tumor, said method comprising delivering the hydrogel composition to a blood vessel within said mammal that is feeding said tumor.

15. The hydrogel composition for use of any one of claims 11-14, wherein said delivery comprises catheter-directed delivery.

## Patentansprüche

1. Eine Hydrogelzusammensetzung, umfassend:
(a) Gelatine;
(b) ein Nanosilikat; und
(c) ein röntgendichtes Kontrastmittel;
wobei das Nanosilikat ein Silikat-Nanoplättchen ist und wobei das röntgendichte Kontrastmittel Tantalteilchen umfasst.

2. Die Hydrogelzusammensetzung nach Anspruch 1, wobei die Hydrogelzusammensetzung etwa 0,1% (Gew./Vol.) bis etwa 20% (Gew./Vol.) der Gelatine umfasst.

3. Die Hydrogelzusammensetzung nach Anspruch 2, wobei die Hydrogelzusammensetzung 18% (Gew./Vol.) der Gelatine umfasst.

4. Die Hydrogelzusammensetzung nach einem der Ansprüche 2-3, wobei die Gelatine eine Gelatine vom Typ A ist.

5. Die Hydrogelzusammensetzung nach Anspruch 1, wobei die Hydrogelzusammensetzung etwa 3% (Gew./Vol.) bis etwa 9% (Gew./Vol.) des Nanosilikats umfasst.

6. Die Hydrogelzusammensetzung nach Anspruch 5, wobei die Hydrogelzusammensetzung etwa 9% (Gew./Vol.) des Nanosilikats umfasst.

7. Die Hydrogelzusammensetzung nach Anspruch 1, wobei die Hydrogelzusammensetzung etwa 2% (Gew./Gew.) bis etwa 30% (Gew./Gew.) des röntgendichten Kontrastmittels umfasst.

8. Die Hydrogelzusammensetzung nach Anspruch 1, wobei die Hydrogelzusammensetzung etwa 20% (Gew./Gew.) des röntgendichten Kontrastmittels umfasst.

9. Die Hydrogelzusammensetzung nach Anspruch 1, umfassend
etwa 15% bis etwa 25% Tantalteilchen (Gew./Gew.);
etwa 2,5% bis etwa 6% Silikat-Nanoplättchen (Gew./Gew.);
etwa 0,2% bis etwa 1,5% Gelatine (Gew./Gew.); und
Wasser.

10. Die Hydrogelzusammensetzung nach Anspruch 1, umfassend
etwa 15% bis etwa 25% Tantalteilchen (Gew./Gew.);
etwa 2,0% bis etwa 5% Silikat-Nanoplättchen (Gew./Gew.);
etwa 0,4% bis etwa 0,8% Gelatine (Gew./Gew.); und
Wasser.

11. Eine Hydrogelzusammensetzung wie in einem der Ansprüche 1-10 definiert zur Verwendung in einem Verfahren zur Embolisation eines Blutgefäßes in einem Säuger, wobei das Verfahren Zufuhr der Hydrogelzusammensetzung zu dem Blutgefäß umfasst.

12. Eine Hydrogelzusammensetzung wie in einem der Ansprüche 1-10 definiert zur Verwendung in einem Verfahren zum Bewirken von Gerinnung in einem Blutgefäß in einem Säuger, wobei das Verfahren Zufuhr der Hydrogelzusammensetzung zu dem Blutgefäß umfasst.

13. Eine Hydrogelzusammensetzung wie in einem der Ansprüche 1-10 definiert zur Verwendung in einem Verfahren zur Behandlung eines Säugers mit einer Blutungsstörung, wobei das Verfahren Zufuhr der Hydrogelzusammensetzung zu einem Blutgefäß in dem Säuger umfasst.

14. Eine Hydrogelzusammensetzung wie in einem der Ansprüche 1-10 definiert zur Verwendung in einem Verfahren zur Behandlung eines Säugers mit einem Tumor, wobei das Verfahren Zufuhr der Hydrogelzusammensetzung zu einem Blutgefäß in dem Säuger, welches den Tumor versorgt, umfasst.

15. Die Hydrogelzusammensetzung zur Verwendung nach einem der Ansprüche 11-14, wobei die Zufuhr kathetergesteuerte Zufuhr umfasst.

## Revendications

1. Composition d'hydrogel comprenant :
(a) de la gélatine ;
(b) un nanosilicate ; et
(c) un agent de contraste radio-opaque ;
dans laquelle ledit nanosilicate est une nanoplaquette de silicate, et dans laquelle ledit agent de contraste radio-opaque comprend des particules de tantale.

2. Composition d'hydrogel selon la revendication 1, dans laquelle ladite composition d'hydrogel comprend d'environ 0,1 % (p/v) à environ 20 % (p/v) de ladite gélatine.

3. Composition d'hydrogel selon la revendication 2, dans laquelle ladite composition d'hydrogel comprend 18 % (p/v) de ladite gélatine.

4. Composition d'hydrogel selon l'une quelconque des revendications 2 à 3, dans laquelle ladite gélatine est une gélatine de type A.

5. Composition d'hydrogel selon la revendication 1, dans laquelle ladite composition d'hydrogel comprend d'environ 3 % (p/v) à environ 9 % (p/v) dudit nanosilicate.

6. Composition d'hydrogel selon la revendication 5, dans laquelle ladite composition d'hydrogel comprend environ 9 % (p/v) dudit nanosilicate.

7. Composition d'hydrogel selon la revendication 1, dans laquelle ladite composition d'hydrogel comprend d'environ 2 % (p/p) à environ 30 % (p/p) dudit agent de contraste radio-opaque.

8. Composition d'hydrogel selon la revendication 1, dans laquelle ladite composition d'hydrogel comprend environ 20 % (p/p) dudit agent de contraste radio-opaque.

9. Composition d'hydrogel selon la revendication 1, comprenant
d'environ 15 % à environ 25 % (p/p) de particules de tantale ;
d'environ 2,5 % à environ 6 % (p/p) de nanoplaquettes de silicate ;
d'environ 0,2 % à environ 1,5 % (p/p) de gélatine ; et
de l'eau.

10. Composition d'hydrogel selon la revendication 1, comprenant
d'environ 15 % à environ 25 % (p/p) de particules de tantale ;
d'environ 2,0 % à environ 5 % (p/p) de nanoplaquettes de silicate ;
d'environ 0,4 % à environ 0,8 % (p/p) de gélatine ; et
de l'eau.

11. Composition d'hydrogel selon l'une quelconque des revendications 1 à 10, pour une utilisation dans une méthode d'embolisation d'un vaisseau sanguin à l'intérieur d'un mammifère, ladite méthode comprenant la délivrance de la composition d'hydrogel audit vaisseau sanguin.

12. Composition d'hydrogel selon l'une quelconque des revendications 1 à 10, pour une utilisation dans une méthode d'induction d'une coagulation dans un vaisseau sanguin à l'intérieur d'un mammifère, ladite méthode comprenant la délivrance de la composition d'hydrogel audit vaisseau sanguin.

13. Composition d'hydrogel selon l'une quelconque des revendications 1 à 10, pour une utilisation dans une méthode de traitement d'un mammifère présentant un trouble hémostatique, ladite méthode comprenant la délivrance de la composition d'hydrogel à un vaisseau sanguin à l'intérieur dudit mammifère.

14. Composition d'hydrogel selon l'une quelconque des revendications 1 à 10, pour une utilisation dans une méthode de traitement d'un mammifère ayant une tumeur, ladite méthode comprenant la délivrance de la composition d'hydrogel à un vaisseau sanguin, qui alimente ladite tumeur, à l'intérieur dudit mammifère.

15. Composition d'hydrogel pour une utilisation selon l'une quelconque des revendications 11 à 14, dans laquelle ladite délivrance comprend une délivrance par cathéter.
